(19) Europäisches Patentamt / European Patent Office / Office européen des brevets

(11) **EP 2 393 525 B1**

(12) **EUROPÄISCHE PATENTSCHRIFT**

(45) Veröffentlichungstag und Bekanntmachung des Hinweises auf die Patenterteilung:
**07.01.2015 Patentblatt 2015/02**

(21) Anmeldenummer: **10704129.5**

(22) Anmeldetag: **08.02.2010**

(51) Int Cl.:
***A61L 27/54*** *(2006.01)* ***A61L 31/16*** *(2006.01)*

(86) Internationale Anmeldenummer:
**PCT/EP2010/051490**

(87) Internationale Veröffentlichungsnummer:
**WO 2010/089389 (12.08.2010 Gazette 2010/32)**

(54) **MIT ALKYLPHOSPHOCHOLINEN BEHANDELTE INTRAOKULARLINSEN ZUR PHARMAKOLOGISCHEN NACHSTARPROPHYLAXE**

INTRAOCULAR LENSES TREATED WITH ALKYLPHOSPHOCHOLINES FOR PHARMACOLOGICAL AFTER-CATARACT PROPHYLAXIS

LENTILLES INTRAOCULAIRES TRAITÉES AVEC DES ALKYL-PHOSPHOCHOLINES POUR UN EFFET PROPHYLACTIQUE DE LA CATARACTE SECONDAIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **06.02.2009 DE 102009007853**

(43) Veröffentlichungstag der Anmeldung:
**14.12.2011 Patentblatt 2011/50**

(73) Patentinhaber:
- **Ludwig-Maximilians-Universität München 80539 München (DE)**
- **Max-Planck-Gesellschaft zur Förderung der Wissenschaften e.V. 80539 München (DE)**

(72) Erfinder:
- **EIBL-LINDNER, Kirsten 81377 München (DE)**
- **KAMPIK, Anselm 81479 München (DE)**
- **EIBL, Hansjörg 37120 Bovenden (DE)**

(74) Vertreter: **Dey, Michael et al Weickmann & Weickmann Patentanwälte Postfach 860820 81635 München (DE)**

(56) Entgegenhaltungen:
**US-A- 5 342 621**
**US-A1- 2003 219 909**
**US-A1- 2008 090 781**

- **SHIGETA ET AL: "Suppression of fibroblast and bacterial adhesion by MPC coating on acrylic intraocular lenses", JOURNAL CATARACT AND REFRACTIVE SURGERY, SURGERY, FAIRFAX, VA, US, Bd. 32, Nr. 5, 1. Mai 2006 (2006-05-01), Seiten 859-866, XP005488905, ISSN: 0886-3350**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft medizinische Materialien und insbesondere ophtalmologische Implantate, welche Alkylphosphocholine (APC) enthalten. Solche Implantate können insbesondere zur Prophylaxe von Nachstar vorteilhaft eingesetzt werden.

US 5,342,621 beschreibt eine antithrombogene Oberfläche. In US 5,342,621 werden medizinische Devices beschrieben, welche Heparin-Phosphatidylcholin (HPTC) enthalten oder mit einem solchen Material beschichtet sind.

Shigeta et al., J. Cataract. Refract. Surg., Vol. 32, May 2006, 859-864 beschreibt die Vermeidung von Fibroblasten- und Bakterienadhäsion an acrylische Intraokularlinsen mittels eines MPC-Coating. Bei MPC handelt es sich um Poly(2-methacryloyloxyethylphosphocholin-co-n-butyl-methacrylat).

US 2003/0219909 A1 beschreibt Materialien, insbesondere ophthalmische Devices, die eine Beschichtung aus Vesikeln enthalten. Bei den Vesikeln kann es sich um Liposomen handeln. Geeignete Materialien zur Bildung der Liposomen können gemäß US 2003/0219909 Phospholipide sein.

US 2008/0090781 A1 beschreibt Formulierungen enthaltend Alkylphosphocholine unter Verwendung von neuen negativen Ladungsträgern.

Der Graue Star oder die Katarakt ist die häufigste Ursache für eine vermeidbare Erblindung im Alter und/oder in Verbindung mit Diabetes mellitus (Klein et al.; Ophthalmology 1984; 91: 1-9) weltweit (48 %; WHO

Global Initiative to Eliminate Avoidable Blindness, "Vision 2020: The Right to Sight"). Sie entsteht durch eine Trübung der Augenlinse, wodurch die Transmission von Licht durch das Auge bis auf die Netzhaut vermindert wird (Medientrübung). Durch eine relativ einfache Operation, die Entfernung der getrübten Linse mit Implantation einer Kunstlinse (Intraokularlinse; IOL) in den verbliebenen Kapselsack, kann den Patienten bei intakter Netzhaut sofort wieder eine gute Sehschärfe ermöglicht werden.

In Deutschland werden nach Schätzungen der Industrie ca. 520 000 Kataraktoperationen pro Jahr durchgeführt (nach "Derzeitiger Stand der Katarakt- und refraktiven Chirurgie - Ergebnisse der Deutschen Gesellschaft für Intraokularlinsenimplantation, Interventionelle und Refraktive Chirurgie DGII sowie des Bundesverbands der Augenärzte BVA - Umfrage 1999"; Ergebnisse der aktuellen Umfrage im Dezember 2008 zu erwarten). In USA werden ca. 1,5 Millionen Kataraktoperationen jährlich durchgeführt (Schein et al. N Engl J Med 2000). Weltweit werden etwa 10 Millionen KataraktOperationen im Jahr durchgeführt.

Die häufigste Komplikation nach Kataraktchirurgie ist die so genannte hintere Kapselfibrose oder der Nachstar (posterior capsule opafication, auch als after-cataract bezeichnet), der eine erneute Minderung der zentralen Sehschärfe verursacht. Der Nachstar tritt bei 11,8 % aller Patienten innerhalb eines Jahres nach operativer Linsenentfernung und Implantation einer IOL auf und bei bis zu 28,4 % aller operierten Patienten nach 5 Jahren. Die höchste Inzidenz wird bei Kindern nach Operation einer angeborenen Katarakt (kongenitale Katarakt) mit über 90 % beobachtet (Kugelberg et al. J Cat Refract Surg 2005, 31: 757-762).

Die Inzidenz hängt des Weiteren von Linsenmaterial und Linsendesign ab: PMMA Linsen zeigen eine Nachstarrate von bis zu 55 %, hydrophobe Akrylate/Silikonlinsen nur von 2,2 %. Akkomodative IOLs wie die 1-CU Linse (HumanOptics AG, Erlangen) sind derzeit jedoch in ihrer Funktion durch die extrem hohe Nachstarrate von 100 % bzw. die dann notwendige Beseitigung stark in ihrer Funktion beeinträchtigt (Mastropasqua et al. Acta Ophthalmol Scand 2007, 85: 409-414). Relativ hohe Nachstarraten von 20 % sind auch für multifokale IOLs beschrieben worden (Meta-Analyse). Mit höheren Nachstarraten sind auch Hydrogellinsen belastet.

Ursache für die Nachstarbildung sind residuale äquatoriale Linsenepithelzellen, die von der Äquatorregion des nach Entfernung der Augenlinse verbliebenen Kapselsackes in die Mitte der optischen Achse wandern (Migration), sich vermehren (Proliferation) und an der Oberfläche der Kunstlinse anhaften (Attachment) mit der Folge einer signifikanten Minderung der zentralen Sehschärfe. Das Oberflächenmaterial der IOL scheint die Linsenepithelzellen zu stimulieren, wodurch Zytokine (IL-1 und 6; PGE2) produziert werden, die neben der oben genannten zellulären Reaktion in einer Störung der Blut-Kammerwasser-Schranke (PGE2 vermittelt) mit inflammatorischer Reaktion resultieren.

Als Goldstandard der Therapie des ausgebildeten Nachstars gilt die Nd:YAG Laser Kapsulotomie. Dies ist ein einfach durchzuführender ambulanter Eingriff, bei dem mittels eines Nd:YAG Lasers die fibrosierte hintere Linsenkapsel eröffnet wird und ein signifikanter Visusanstieg erfolgt. Allerdings betragen die jährlichen Kosten in den USA für diese Art der Therapie allein ca. 250 Millionen USD. Außerdem sind moderne IOLs wie die akkomodativen und multifokalen Linsen in ihrer Funktionsfähigkeit durch den Lasereingriff stark beeinträchtigt: Akkomodative IOLs verlieren ihre Akkomodationsfähigkeit (Mastropasqua et al. Acta Ophthalmol Scand 2007, 85: 409-414) und multifokale IOLs können durch eine Dezentrierung der Linse im Kapselsack in ihrer Abbildungsqualität leiden. Zudem ist der Zugang zu einer Nd:YAG Kapsulotomie nicht in allen Teilen der Welt gleichermaßen gewährleistet.

Zudem treten häufig Komplikationen bei der Laserbehandlung auf, wie z.B. eine Beschädigung der IOL durch den Laser (12 %), ein sekundärer Druckanstieg (Sekundärglaukom; 8,5 %) oder eine Netzhautschädigung (ca. 1 %) sowie eine IOL Subluxation oder Dislokation (0,10 %).

Wünschenswert wäre für die Qualität der ophthalmologischen Versorgung weltweit sowie aus Gründen der

Kosteneffektivität deshalb eine prophylaktische Maßnahme, die einen Nachstar gar nicht erst entstehen lässt. Erwiesenermaßen prophylaktisch wirksam ist bisher nach Meta-Analyse jedoch nur ein IOL Optikdesign mit scharfer Optikkante ("sharp edge design"; Findl et al. Cochrane Database of Systematic Reviews 2007) im Vergleich zu Linsen mit runder Optikkante.

Trotz der Verwendung von IOLs mit scharfer Optikkante hat sich jedoch nach aktueller statistischer Auswertung der Daten von US-amerikanischen Versicherungsgesellschaften und deren Ausgaben nicht die erhoffte Kostenreduktion durch dieses Linsendesign ergeben (Cleary und Spalton, ESCRS Eurotimes 2008, 115: 1308-1314). Ursache hierfür könnten die bisher eher kurzen Nachbeobachtungszeiten von zum Teil nur drei Jahren nach Kataraktoperation/Linsenimplantation sein (Kohnen et al. Ophthalmology 2008). Außerdem ist fraglich, inwiefern die oben genannten technischen Vorgaben hinsichtlich Linsenmaterial und -design zur Senkung der Nachstarinzidenz auch für die Konzeption von IOLs der neuen Generation, also akkomodativen und multifokalen IOLs, technisch umzusetzen sind.

Modifikationen der chirurgischen Technik sowie die intra- oder perioperative Applikation eines Medikaments zur pharmakologischen Nachstarprophylaxe haben sich bisher als nicht signifikant auf die Nachstarentwicklung erwiesen.

Die pharmakologischen Ansätze zur Nachstarprophylaxe sind zahlreich, jedoch im klinischen Kontext bisher nicht erfolgreich. Folgende pharmakologische Substanzen wurden bisher getestet: Mitomycin C (MMC; Chung et al. J Cataract Refract Surg 2000), Ethylendiamintetraessigsäure (EDTA; Nishi et al. J Cataract Refract Surg 1999), 5-Fluoruracil (5-FU; Ruiz et al. Ophthalmic Res 1990) und Daunomycin (Power et al. J Cataract Refract Surg 1994) sowie eine Vielzahl weiterer, wie z.B. Zytostatika, darunter Antimetobolite wie Methotrexat oder 5-FU, Antibiotika wie Daunomycin oder Mitomycin, Antimitotika wie Colchizin; NSAR wie Indomethazin oder Diclofenac und andere wie Heparin, Sumarin oder Transferrin. Aufgrund fehlender Effektivität bzw. toxischer Nebenwirkungen ist der klinische Einsatz dieser Substanzen limitiert (Pilotstudien). Effektive Substanzen wie Mitomycin C (Kim et al. Clin Exp Ophthalmol 2007), Thapsigargin / 5-Fluorouracil (Abdelwahab et al. Eye 2008) und Triton X-100/destilliertes Wasser (Maloof et al. Arch Ophthalmol 2005) erfordern während der Applikation im Auge die Verwendung eines Spülsystems in der geschlossenen Kapsel ("sealed-capsule irrigation device"), um die signifikanten toxischen Nebenwirkungen der Substanzen zu verhindern. Dies bedeutet einen deutlichen operativen Mehraufwand sowie längere Operationszeiten. Destilliertes Wasser (im o.g. "sealed-capsule device" angewandt) allein hat sich jedoch als nicht effektiv erwiesen, wie in 17 Patienten nach zwei Jahren Nachbeobachtungszeit gezeigt werden konnte (Rabsilber et al. Br J Ophthalmol 2007). Substanzen wie Heparin auf Oberflächen-modifizierten hydrophilen Acryl-IOLs (BioVue®, OII, Ontario, CA, USA) sind unwirksam hinsichtlich ihrer prophylaktischen Wirkung auf die Nachstarentstehung im Vergleich zu hydrophoben Acryl-IOLs (sensor®, AR 40e, AMO, Santa Ana, CA, USA), wie in einer aktuellen randomisierten klinischen Studie an hundert Patienten gezeigt wurde (Kang et al. Eur J Ophthalmol 2008). Zudem ist bei intraokularer Anwendung von Heparin aufgrund seiner Blut verdünnenden Eigenschaften mit einem Blutungsrisiko zu rechnen.

Bei den bisher zur Nachstarprophylaxe eingesetzten Substanzen handelt es sich oftmals um Antitumormittel mit unangenehmen und nicht erwünschten Nebenwirkungen, welche pharmazeutisch gut beschrieben und gut charakterisiert sind. Zudem sind die bisher eingesetzten Substanzen zumeist sehr gut wasserlöslich und haften aufgrund ihrer guten Wasserlöslichkeit weder auf hydrophilen noch auf hydrophoben Materialien.

Eine Aufgabe der vorliegenden Erfindung bestand deshalb darin, medizinische Materialien mit einem günstigen Einfluss auf den Wundheilungsprozess bereitzustellen.

Diese Aufgabe wird erfindungsgemäß gelöst durch ein medizinisches Material, welches dadurch gekennzeichnet ist, dass es eine Alkylphosphocholin-Verbindung der Formel (I)

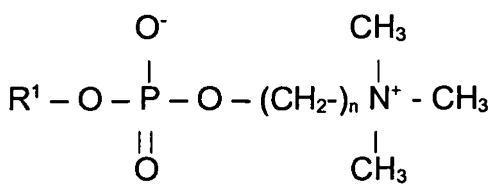

(I)

enthält, worin

$R^1$ ein gegebenenfalls Heteroatome enthaltender $C_{16}$-$C_{24}$-Kohlenwasserstoffrest ist und
n eine ganze Zahl von 1 bis 5 darstellt.

Es wurde festgestellt, dass das erfindungsgemäße medizinische Material enthaltend eine Alkylphosphocholin-Verbindung aufgrund seiner Oberflächeneigenschaften und seiner Biokompatibilität eine Verbesserung der Wundheilungsreaktion und eine Verminderung von Fremdkörperreaktionen bewirkt. Das medizinische Material ist dabei vorzugsweise mit der Alkylphosphocholin-Verbindung beschichtet. Es ist aber auch möglich, die Alkylphosphocholin-Verbindung dem Grundmaterial des medizinischen Materials beizumischen.

In einer bevorzugten Ausführungsform wird die Alkylphosphocholin-Verbindung durch Eintauchen des medizinischen Materials in eine Lösung der Alkylphosphocholin-Verbindung aufgebracht.

Erfindungsgemäß wurde überraschenderweise festgestellt, dass medizinische Materialien mit Alkylphosphocholin-Verbindungen beschichtet werden können. Dabei kann die Beschichtung direkt auf einfache Weise aufgebracht werden, ohne dass Hilfssubstanzen, Linker, sonstige Verknüpfungssubstanzen oder Vorbeschichtungen erforderlich sind.

Bei dem erfindungsgemäßen medizinischen Material handelt es sich insbesondere um ein inertes biokompatibles Material, vorzugsweise um ein inertes biokompatibles festes Material. Bevorzugte medizinische Materialien sind beispielsweise Implantate oder medizinische Vorrichtungen.

Bei dem erfindungsgemäßen medizinischen Material handelt es sich insbesondere um ein polymeres Material, aus dem dann medizinische Vorrichtungen oder Implantate gebildet werden können. Geeignete polymere Materialien sind bespielsweise hydrophile oder hydrophobe Polymere, wobei hydrophile Polymere bevorzugt sind. Beispiele für solche polymere Materialien sind unter anderem Acrylate, insbesondere hydrophobes Acrylat oder hydrophiles Acrylat, Methacrylate, insbesondere Polymethylmethacrylat (PMMA) oder Silikone.

Bei dem Material kann es sich aber auch um ein Metall oder eine Metalllegierung handeln, umfassend z.B. Titan, Eisen oder/und Cobalt.

Das erfindungsgemäße medizinische Material kann insbesondere zur Herstellung von medizinischen Vorrichtungen oder Implantanten verwendet werden. Deshalb umfasst die Erfindung auch medizinische Implantante oder in der Diagnose, Therapie, Prophylaxe oder/und zur Verbesserung von unerwünschten Zuständen eingesetzten medizinischen Materialien, welche wenigstens teilweise aus dem erfindungsgemäßen medizinischen Material gebildet sind. Vorzugsweise wird zumindest der mit dem Körper oder Körperflüssigkeiten in Kontakt kommende Teil aus dem erfindungsgemäßen medizinischen Material gebildet. Es ist aber auch möglich, ein vollständiges medizinisches Implantat oder Material aus dem erfindungsgemäßen medizinischen Material bereitzustellen.

Materialien, die wenigstens zum Teil aus dem erfindungsgemäßen medizinischen Material gebildet werden, sind beispielsweise Stents, Herzklappen, Verweilkatheter, Prothesen, Implantante oder/und Nahtmaterialien oder/und Kontaktlinsen.

Aufgrund der grenzflächenaktiven Eigenschaften der Phosphocholine und ihrer Tendenz, an Grenzflächen monomolekulare Filme auszubilden, können verschiedenste Materialien mit Phosphocholinen beschichtet werden.

Eine Aufgabe der vorliegenden Erfindung bestand insbesondere darin, ophthalmologische Implantate bereitzustellen, durch die eine Nachstarentstehung verhindert oder zumindest vermindert wird.

Diese Aufgabe wird erfindungsgemäß gelöst durch ein ophthalmologisches Implantat, welches dadurch gekennzeichnet ist, dass es ein Alkylphosphocholin enthält.

Es wurde festgestellt, dass die erfindungsgemäßen ophthalmologischen Implantate eine effektive, nicht-toxische Möglichkeit der pharmakologischen Nachstarprophylaxe liefern. Im Gegensatz zu vielen bisherigen Ansätzen wird erfindungsgemäß der Wirkstoff nicht nur separat, beispielsweise durch topische Applikation, verabreicht, sondern stellt einen Bestandteil des ophthalmologischen Implantats dar. Der Wirkstoff kann dabei in das Implantatmaterial inkorporiert sein. Oftmals ist es jedoch besonders vorteilhaft, Implantate mit einer Oberflächenbeschichtung aus Alkylphosphocholinen bereitzustellen. Es wurde festgestellt, dass mit Alkylphosphocholinen die Oberflächen von ophthalmologischen Implantaten, insbesondere von Intraokularlinsen, modifiziert werden können. Dies war überraschend, da eine Beschichtung von ophthalmologischen Implantaten, insbesondere von Intraokularlinsen mit vielen Substanzen nicht möglich ist.

Es konnte nun gezeigt werden, dass mit Alkylphosphocholinen ophthalmologische Impantate und insbesondere Intraokularlinsen beschichtet werden können. Dies erleichtert die Anwendung deutlich. Insbesondere ergeben sich bei dieser Vorgehensweise keine längeren Operationszeiten durch eine intraokulare Applikation einer Wirksubstanz. Vielmehr kann das bereits mit den Alkylphosphocholinen (APC) Oberflächen-modifizierte Implantat, insbesondere eine Intraokularlinse, wie sonst auch üblich ohne operative Zusatzmaßnahmen direkt implantiert werden.

Überraschenderweise wurde nun festgestellt, dass durch ophthalmologische Implantate, welche Alkylphosphocholine enthalten, insbesondere durch mit Alkylphosphocholinen beschichteten Intraokularlinsen, erfolgreich ein Auftreten von Nachstar vermindert werden kann. Dies ist im Gegensatz zu anderen zuvor in in vitro getesteten proliferationshemmenden Wirkstoffen, die keine prophylaktische Wirkung als Beschichtung auf Intraokularlinsen zeigen.

Weiterhin wurde erfindungsgemäß festgestellt, dass APCs auch im hinteren Augenabschnitt gut verträglich sind und insbesondere keine retinale Toxizität induzieren. Dies ist wichtig, da es bei der operativen Entfernung einer Augenlinse auch zu einem Riss in der hinteren Linsenkapsel kommen kann und so eine Verbindung zwischen vorderem und hinterem Augenabschnitt entstehen kann (durch Wegfall der Diffusionsbarriere).

In einer besonders bevorzugten Ausführungsform handelt es sich bei dem erfindungsgemäßen ophthalmologischen Implantat um eine Intraokularlinse. Es kann sich aber auch um andere Implantate, beispielsweise um ein refraktives, verformbares Polymer handeln, wie es bei der refraktiven Linsenchirurgie, dem sogenannten "lens refilling" (Nishi et al., J. Cat. Refract. Surg. 1998 und 2008; Koopmans et al., Invest Ophthalmol Vis Sci 2003 und 2006) zum Einsatz kommt. In diesem Anwendungsbereich wird das Polymer vorzugsweise mit dem Alkylphosphocholin vermischt.

Durch das Inkorporieren bzw. Beschichten eines ophthalmologischen Implantats mit einem Alkylphosphocholin wird die Oberfläche des Implantats modifiziert.

Besonders bevorzugt wird erfindungsgemäß die Oberfläche eines ophthalmologischen Implantats, insbesondere die Oberfläche von Intraokularlinsen, mit einem Alkylphosphocholin, insbesondere Oleylphosphocholin, Erucylphosphocholin (ErPC) oder Erucylphosphohomocholin (Erufosin; $ErPC_3$) beschichtet, insbesondere unter Ausbildung eines monomolekularen Oberflächenfilms.

Bei den erfindungsgemäß einsetzbaren Alkylphosphocholin-Verbindungen handelt es sich insbesondere um lipophile Alkylphosphocholin-Verbindungen. Solche lipophile Alkylphosphocholin-Verbindungen weisen vorzugsweise wenigstens einen Kohlenwasserstoffrest, insbesondere einen gesättigten Alkylrest oder einen Kohlenwasserstoffrest, der ein oder mehr ungesättigte Doppelbindungen enthält, mit mindestens 16 C-Atomen auf. Durch den Einsatz von lipophilen Alkylphosphocholinen wird eine einfache Beschichtung von medizinischen Materialien, insbesondere von ophthalmologischen Implantaten ermöglicht.

Vorzugsweise wird eine Phosphocholin-Verbindung eingesetzt, die grenzflächenaktiv ist und insbesondere eine pharmazeutische Wirkung besitzt und besonders bevorzugt antiproliferativ, antiparasitär, antimykotisch oder/und antibakteriell wirkt. Vorzugsweise wird eine Phosphocholin-Verbindung mit antiproliferativer Wirkung eingesetzt.

Erfindungsgemäß bevorzugt weist die Alkylphosphocholin-Verbindung die Formel (I) auf

$$R^1\text{-O-}PO_2^-\text{-O-}(CH_2\text{-})_n\text{-}N^+(CH_3)_3$$

worin $R^1$ ein gegebenenfalls Heteroatome enthaltender $C_{16}$-$C_{24}$-Kohlenwasserstoffrest ist und n eine ganze Zahl von 1 bis 5 darstellt. Besonders bevorzugt ist n 2 oder 3. $R^1$ ist insbesondere ein $C_{16}$-$C_{24}$-Kohlenwasserstoffrest. $R^1$ kann ein gesättigter Alkylrest sein, er kann aber auch ein, zwei, drei oder mehr ungesättigte Bindungen, insbesondere Cis-Doppelbindungen enthalten. Besonders bevorzugt sind Kohlenwasserstoffreste, die wenigstens eine Cis-Doppelbindung enthalten, wobei es sich bei dem Rest $R^1$ besonders bevorzugt um einen Erucyl ($C_{22:1\text{-cis:}\omega\text{-9-Rest)}}$- oder einen Oleyl ($C_{18:1\text{-cis-w-9-Rest}}$)-Rest handelt.

Erfindungsgemäß ist die Phosphocholin-Verbindung ein Alkylphosphocholin der Formel (I)

$$R^1\text{-O-}PO_2^-\text{-O-}(CH_2\text{-})_n\text{-}N^+(CH_3)_3$$

worin $R^1$ ein $C_{16}$-$C_{24}$-Kohlenwasserstoffrest ist und n eine ganze Zahl von 1 bis 5 darstellt. Besonders bevorzugt ist n 2 oder 3.

$R^1$ ist mehr bevorzugt ein $C_{18}$-$C_{24}$ Kohlenwasserstoffrest. Dieser Rest kann ein Alkylrest sein, er kann aber auch ein oder mehrere ungesättigte Bindungen enthalten. Besonders bevorzugt sind Kohlenwasserstoffreste, die wenigstens eine Cis-Doppelbindung enthalten. Besonders bevorzugt handelt es sich bei dem Rest $R^1$ um einen Erucyl ($C_{22}$) oder einen Oleyl ($C_{18}$)-Rest.

Es handelt sich bei der Phosphocholin-Verbindung um ein Alkylphosphocholin. Vorzugsweise handelt es sich nicht um ein Lecithin.

Am meisten bevorzugt handelt es sich bei der Phosphocholin-Verbindung um Erucylphosphocholin, Erucylphosphohomocholin oder Oleylphosphocholin.

Die erfindungsgemäß bevorzugt eingesetzten Substanzen mit einer Alkylkettenlänge von 16 bis 24 Kohlenstoffatomen sind in Wasser nicht gelöst oder monodispers verteilt. Sie bilden in Wasser viel mehr Überstrukturen aus und lagern sich leicht in Grenzflächen oder Membranen ein. Phosphocholin-Verbindungen bilden somit eine Beschichtung auf Materialien, insbesondere auf medizinischen Materialien wie medizinischen Implantanten, vorzugsweise auf ophthalmologischen Implantanten und insbesondere auf Intraokularlinsen. Weiterhin lagern sich Phosphocholine leicht in Polymere mit hydrophoben Grenzflächen oder Oberflächen ein.

Die Beschichtung von medizinischen Materialien, insbesondere von ophthalmologischen Implantanten, mit einer Alkylphosphocholin-Verbindung erfolgt vorzugsweise unter Ausbildung eines monomolekularen Oberflächenfilms.

Alkylphosphocholine (APCs) sind effektive Inhibitoren der okulären Zellproliferation, Migration und Anheftung in nicht-toxischen Konzentrationen. Als synthetische Phospholipid-Derivate repräsentieren sie eine neue Klasse pharmakologisch aktiver Substanzen (Eibl H et al. Cancer Treat Rev 1990) und befinden sich aufgrund ihrer guten antitumoralen (Leonard et al. J Clin Oncol 2001; Miltex®, Zentaris GmbH, Frankfurt) und antiparasitären (Sundar et al. N Engl J Med 2002; Impavido®, Zentaris GmbH, Frankfurt) Eigenschaften erfolgreich im klinischen Einsatz.

Weiterführende *in vivo* Studien an entsprechenden Tiermodellen am Auge haben eine gute Effektivität und Verträglichkeit bei lokaler Gabe mittels intravitrealer Applikation (Injektion in den Glaskörperraum, also in den hinteren Augenabschnitt) gezeigt. So konnte am Rattenauge sieben Tage nach einmaliger Injektion von Erucylphosphocholin (ErPC) in den Glaskörperraum weder morphologisch mittels Licht- und Elektronenmikroskopie noch funktionell nach Ableitung eines Elektroretinogramms (ERG) eine Beeinträchtigung der Netzhautfunktion nachgewiesen werden (Schüttauf et al. Curr Eye Res 2005). Am Kaninchenauge konnte gezeigt werden, dass liposomales ErPC sowie freies $ErPC_3$ einen Tag nach experimentell induzierter Netzhautablösung einmalig intravitreal gegeben eine signifikante Hemmung der intraretinalen Proliferation von Müller Gliazellen sowie von subretinalen Pigmentepithelzellen erzielt ohne morphologisch nachweisbare toxische Nebenwirkungen (Eibl et al. IOVS 2007 und Der Ophthalmologe 2007).

Langzeituntersuchungen zur Verträglichkeit von ErPC am Katzenauge haben 28 Tage nach intravitrealer Gabe ebenfalls keinen Hinweis auf eine retinale Toxizität ergeben. Funktionelle Analysen an einem Retina *ex vivo* Modell haben bis zu einer Konzentration von 25 $\mu$M in der Gesamtlösung keine Veränderungen im Elektroretinogramm von mit $ErPC_3$ inkubierter Netzhaut der Ratte ergeben (siehe auch Beispiel 5 hierin). Vergleichbare Analysen an diesem Modell haben für intraokular andere Substanzen wie Triamcinolon eine retinale Toxizität in klinisch angewandten Dosierungen (4 mg/ml) nachgewiesen (Lüke et al. Exp Eye Res 2008).

Erfindungsgemäß wurde nun festgestellt, dass zusätzlich zur hohen Effektivität und dem Fehlen von toxischen Nebenwirkungen APCs geeignet sind, in ophthalmologische Implantate eingebaut zu werden bzw. ophthalmologische Implantate damit zu beschichten.

Die Möglichkeit, eine Intraokularlinse mit APCs zu beschichten, ist die ideale technische Umsetzung oben genannter Vorkenntnisse in den klinischen Kontext und daher von klinischer Relevanz.

Eine weitere Möglichkeit der Anwendung liegt im Bereich des sogenannten "lens refilling". Hierbei wird die Augenlinse operativ entfernt und ein Polymer unterschiedlicher Zusammensetzung in den Kapselsack eingefüllt. Dieses Polymer dient als elastischer, potentiell verformbarer Ersatz für die getrübte, "steife" Linse des älteren Patienten, die ihre Verformbarkeit (Akkomodationsfähigkeit) und somit Lesefähigkeit verloren hat. Bei vielen Patienten beginnt diese sogenannten Alterssichtigkeit (Presbyopie) bereits mit dem 45. Lebensjahr, so dass sie auf eine Lesebrille angewiesen sind. Durch einen refraktiven Linsenaustausch und die Implantation einer akkomodativen Linse bzw. Multifokallinse oder den neuen Ansatz des "lens refilling" könnten diese Patienten die Lesebrille umgehen, die von Vielen als Makel empfunden wird. Das injizierte Polymer hat ähnliche optische Eigenschaften wie die natürliche, verformbare Linse des jungen Menschen. Hauptproblem ist jedoch die Ausbildung eines Nachstars im postoperativen Verlauf, wodurch sich die Sehschärfe erneut verschlechtert. APCs bieten aufgrund ihrer chemischen Struktur die Möglichkeit, mit dem Polymer gemischt zu werden und so als Bestandteil der neuen Linse in das Auge eingebracht zu werden und dort zu verbleiben.

Ophthalmologische Implantante bestehen zumeist aus Materialien wie Acrylatmaterialien, Silikonmaterialien oder Hydrogelen, insbesondere hydrophobe Acrylate, hydrophile Acrylate, Silikon und vorzugsweise PMMA.

Erfindungsgemäß bevorzugt sind beispielsweise hydrophobe Intraokularlinsen, welche aus Acrylat oder Silikon gebildet sein können oder hydrophile Intraokularlinsen, welche aus Acrylat gebildet sind.

Eine Beschichtung von solchen Materialien gestaltet sich äußerst schwierig. Ein Grund dafür ist die hohe Wasserlöslichkeit der verwendeten Wirkstoffe, wie etwa 5-Fluoruracil, Methotrexat, Triton-X-100. Insbesondere erfolgt oftmals eine Ablösung der Wirkstoffe, sobald die Intraokularlinsen mit einem wässrigen Medium, wie es im Auge vorliegt, in Berührung kommen.

Es wurde nun festgestellt, dass solche Implantatmaterialien hervorragend mit Alkylphosphocholinen beschichtet werden können bzw. dass Alkylphosphocholine in Implantatmaterialien ohne Weiteres inkorporiert werden können.

Insbesondere lipophile Alkylphosphocholin-Verbindungen eignen sich hervorragend für die Beschichtung von Implantat-Materialien. Dabei können die Alkylphosphocholin-Verbindungen direkt ohne Hilfssubstanzen, ohne Linker und ohne die Erforderlichkeit einer Vorbeschichtung oder sonstiger Maßnahmen direkt als Beschichtung auf die Implantat-Materialien aufgebracht werden.

Alkylphosphocholine, wie hierin beschrieben, besitzen besondere Eigenschaften und bilden in Wasser Überstrukturen, sogenannte Mizellen, aus, die durch die kritische Mizell-Konzentration gut charakterisiert werden können. Diese Mizellen lagern sich in lipophile Oberflächen und Grenzflächen spontan ein und können durch wässrige Medien nicht oder nur schwer wieder entfernt werden, im Gegensatz zu wasserlöslichen Wirkstoffen, die leicht abgelöst werden können.

Erfindungsgemäß ist festgestellt worden, dass eine Beschichtung von medizinischen Materialien und insbesondere von ophthalmologischen Implantanten mit Alkylphosphocholinen auf einfache Weise durchgeführt werden kann.

Es wurde insbesondere festgestellt, dass zur Beschichtung von intraokularen Implantanten mit APCs keine zusätzlichen Hilfssubstanzen, Linker oder andere Modifikationen der Oberflächen oder des Materials, beispielsweise von Linsen, erforderlich ist. Vielmehr können die APCs direkt, z.B. unmittelbar aus der Lösung, beispielsweise aus einer wässrigen, z.B. aus einer physiologischen

Kochsalzlösung oder aus Wasser, oder einer alkoholischen, insbesondere einer ethanolischen oder methanolischen Lösung direkt auf die Oberfläche des medizinischen Materials als Beschichtung aufgebracht werden, oder in das medizinische Material eingebracht werden. Selbst bei mehrmaligem Waschen mit wässrigen Lösungen, beispielsweise mit wässriger physiologischer Kochsalzlösung, bleiben die APCs auf der Oberfläche des ophthalmologischen Implantants haften.

Die Menge an inkorporierter beziehungsweise aufgebrachter APC-Verbindung beträgt dabei vorteilhafterweise von 0,1 bis 200 $\mu$M, insbesondere von 1 bis 100 $\mu$M APC, bezogen auf die Masse des ophthalmologischen Implantats bzw. 1 bis 600 pmol, vorzugsweise 2 bis 100 pmol und inbesondere 4 bis 8 pmol APC, pro $mm^2$ Oberfläche des medizinischen Implantats, z.B. eines ophthalmologischen Implantats, insbesondere eine monomolekulare Schicht an APC, auf der Oberfläche.

Weiterhin wurde festgestellt, dass mit APCs beschichtete ophthalmologische Implantante, beispielsweise Intraokularlinsen, eine hervorragende Wirksamkeit zur Prophylaxe von Nachstar aufweisen. Die Intraokularimplantate entfalten ihre pharmakologische Wirkung insbesondere nicht nur sofort nach der Implantation, sondern weisen eine längerfristige prophylaktische Wirkung auf, insbesondere auf die zelluläre Proliferation, Migration und Anheftung, also auf den Wundheilungsprozess insgesamt. Dies ist gerade zur Verhinderung der Entstehung von Nachstar von größter Relevanz, da sich dieser Prozess langsam über bis zu 5 Jahre postoperativ entwickeln kann. Dabei schreiten die im Kapselsack verbliebenen Linsenepithelien langsam in die optische Achse und führen oftmals erst Monate oder Jahre nach der Operation zu entsprechenden Beschwerden von Seiten des Patienten, wie etwa Nebelsehen, dunkler oder unscharf Sehen, Blendungsgefühl oder Beschwerden beim Lesen. Durch erfindungsgemäß beschichtete

Intraokularlinsen kann auch langfristig eine Entstehung des Nachstars wirksam verhindert werden.

Zusammenfassend kann festgestellt werden, dass Alkylphosphocholine aufgrund ihrer antiproliferativen Eigenschaften, ihrer guten Verträglichkeit sowie ihrer Kompatibilität mit ophthalmologischen Implantatmaterialien hervorragend zur Anwendung in der pharmakologischen Nachstarprophylaxe geeignet sind. Die Intraokularlinsen sind mit bloßem Auge nicht von unbeschichteten Kontroll-Intraokularlinsen der gleichen Charge zu unterscheiden. Von besonderem Interesse ist die Oberflächenmodifikation von Intraokularlinsen mit Alkylphosphocholinen.

Aufgrund der guten Verträglichkeit sind bei Alkylphosphocholinen operative Zusatzmaßnahmen, wie beispielsweise die Verwendung eines "sealed capsule"-Systems nicht erforderlich.

Als Phosphocholin-Verbindungen werden die hierin zuvor erläuterten, $C_{16}$-$C_{24}$-Alkylphosphocholine eingesetzt.

Es wurde festgestellt, dass sich Phosphocholin-Verbindungen insbesondere zur Behandlung der folgenden ophthalmologischen Erkrankungen hervorragend eignen:

- Lid- oder Okularadnexe, insbesondere bei Infektion, Inflammation, postoperative Zustände, Narben, Trauma, kosmetische Indikationen, Malignanz, proliferative Erkrankungen, Hautläsionen.
- Erkrankungen der Konjunktiva und/oder der Okularoberfläche, insbesondere Infektion, Inflammation, postoperative Zustände, Narben, Traumata, kosmetische Indikationen, Malignanz, proliferative Erkrankungen, Kontaktlinsen-verursachte Zustände, Lubrikation.
- Erkrankungen der Cornea und/oder der Okularoberfläche, insbesondere Infektion, Inflammation, postoperative Zustände, Narben, Traumata, kosmetische Indikationen, Malignanz, proliferative Erkrankungen, Kontkatlinsen-verursachte Zustände, Lubrikation, Sicca-Syndrom.
- Verwendung als Post-Trabekulektomiemittel, insbesondere als Antinarbenmittel.
- Zur medizinischen Behandlung von Glaukoma, zur IOD-Verringerung oder/und zur Erleichterung des Kammerwasserabflusses.
- Bei Kataraktoperationen zur Verhinderung von Nachstar.
- Bei Netzhauterkankungen, proliferativer Vitreoretinopathie, Ablösung der Retina, diabetischer Retinopathie (NPDR; PRD; Maculopathie), altersbedingter Makuladegeneration, Makulaerkrankung (RCS; CNV).
- Bei Uveitis, bei intraokularer Infektion, bei intraokularer Entzündung, bei intraokularer Malignanz oder/und bei Traumata.

Der Einsatz von Phosphocholin-Verbindungen bei ophthalmologischen Erkrankungen kann auf verschiedene Weise erfolgen, vorzugsweise durch Oberflächenmodifikation und Intraokularlinsen, als Beschichtungsmittel oder/ und Bestandteil von Kontaktlinsen, als Zugabe zu Polymeren für den intraokularen Gebrauch (z.B. lens refilling), als Zugabe oder Beschichtung von intraokularen Vorrichtungen, oder als Zugabe oder Beschichtung von Systemen mit verzögerter Freisetzung.

Ein weiteres Problem, welches oftmals nach einer Katarakt-Operation auftritt, ist das sogenannte "Lens Glistening", also Glitzereffekte auf der Linse. Solche störende Glitzereffekte treten bei etwa 40 bis 67 % aller Patienten auf, die bei einer Katarakt-Operation eine hydrophobe, faltbare Acryllinse implantiert bekommen. Es wurde nunmehr festgestellt, dass durch Beschichtung solcher Linsen mit Alkylphosphocholinen die Bildung dieser Glitzereffekte bzw. Glitzerbläschen verhindert oder zumindest vermindert wird. Dadurch kann die Biokompatibilität solcher Linsen verbessert werden. Ein weiterer Gegenstand der Erfindung ist deshalb die Verwendung von Phosphocholin-Verbindungen zur Verminderung von Glitzereffekten auf Intraokularlinsen. Es wird davon ausgegangen, dass Alkylphosphocholine Glitzereffekte, die durch Berührung der hydrophoben Linsenoberfläche mit dem hydrophilen Kammerwasser entstehen, abschwächen können.

Die Erfindung wird durch die beigefügten Figuren und die folgenden Beispiele weiter erläutert.

**Figur 1** zeigt schematisch die Pathogenese des Nachstars (Nishi et al., J Cat Refract Surg 1996).

**Figur 2** zeigt die Ergebnisse der Zelllebensfähigkeituntersuchung bestimmt durch den Trypanblau-Ausschlusstest und den Lebend-Tot-Assay, wobei kein Unterschied zwischen APC-behandelten und Kontrollzellen festzustellen ist.

**Figur 3** zeigt die konzentrationsabhängige Inhibition von humaner Linsenepithelzellproliferation durch APCs.

**Figur 4** zeigt die dosisabhängige Inhibition von Zellanhaftung von humanen Linsenepithelzellen durch APCs.

**Figur 5** zeigt die dosisabhängige Inhibition der Migration von humanen Linsenepithelzellen.

**Figur 6** zeigt die Inhibition der humanen Linsenepithelproliferation durch Oleylphosphocholin-beschichtete Intraokularlinsen in Form eines Säulendiagramms.

Es zeigt sich, dass Intraokularlinsen aus verschiedenen Materialien (hydrophobe Acrylate, hydrophile Acrylate und Silikone) mit unterschiedlichen Haptik-Designs und Optikdurchmessern mit Alkylphosphocholinen beschichtet (OIPC) das Zellwachstum von bereits proliferierenden Linsenepithelzellen hemmen können.

Zur Bestimmung der Zellzahl wurde ein Tetrazoliumreduktionsassay [MTT (3-[4,5-dimethylthiazol-2-yl]-2,5-diphenyltetrazolium bromide]; (Sigma-Aldrich) durchgeführt. Die Intraokularlinse wurde entnommen, die Zellen wurden mit PBS gewaschen und mit dem MTT Reagenz für 1 Stunde bei 37 °C inkubiert. Nur vitale Zellen verstoffwechseln die Lösung. Anschließend wurde die Zellmembran mittels DMSO (Dimethylsulfoxid) gelöst und die optische Dichte im ELISA-Reader bei 550 nm gemessen. Die optische Dichte korreliert hierbei mit der Anzahl der proliferierenden Zellen.

**Figur 7** zeigt die Inhibition der humanen Linsenepithelproliferation durch Erucyl-Phosphohomocholin (ErPC3)-beschichete Intraokularlinsen in Form eines Säulendiagramms.

Es zeigt sich, dass Intraokularlinsen aus verschiedenen Materialien (hydrophobe Acrylate, hydrophile Acrylate und Silikone), mit unterschiedlichen Haptik-Designs und Optikdurchmessern mit Alkylphosphocholinen beschichtet ($ErPC_3$) das Zellwachstum von bereits proliferierenden Linsenepithelzellen hemmen können. Die Bestimmung erfolgte wie bei Figur 6 beschrieben.

**Figur 8** zeigt humane Linsenepithelzellen in der Zellkultur. Die Aufnahmen erfolgten bei 100-facher Vergrößerung im Phasenkontrastmikroskop.

**Figur 8a** zeigt Linsenepithelzellen neben einer unbeschichteten Kontoll-IOL (links) und neben einer mit APC beschichteten IOL (rechts) nach 48 Stunden.

**Figur 8b** zeigt Linsenepithelzellen neben einer unbeschichteten Kontroll-IOL (links) und neben einer mit APC beschichteten IOL (rechts) nach 48 Stunden.

**Figur 8c** und **Figur 8d** zeigen die Effekte jeweils nach 72 Stunden.

Eine mit Alkylphosphocholin-beschichtete Intraokularlinse führt, wenn sie in Kultur eingebracht wird, zu einer Inhibition der zellulären Ausbreitung und des Wachstums der Zellen sowohl neben als auch unter der Intraokularlinse. Der Effekt verstärkt sich über die Zeit (Zunahme des Effektes von 48 auf 72 Std nach Einbringen der Alkylphosphocholin beschichteten Intraokularlinsen in die Zellkultur).

**Figur 9** zeigt die Biokompatibilität der Alkylphosphocholine am humanen Hornhautendothel am vorderen Augenabschnitt.

Nach Durchführung einer Live/Dead Färbung sind bei Alkylphosphocholin Konzentrationen bis 1 mM keine toten Endothelzellen zu sehen (rote Färbung der Zellen durch Propidiumiodid).

Es wurde ein Live/Dead Test durchgeführt. Zellkerne nichtvitaler Zellen färbten sich hierbei rot, da Propidiumiodid nur in tote Zellen eindringen kann. Zellkerne vitaler Zellen werden mit dem Membran-gängigen Farbstoff Hoechst 33342 (Intergen) blau angefärbt. Zuvor wurden die Zellen auf Vier-Kammer-Trägern ausgesät und mit den APC-Konzentrationen 100 $\mu$M (APC niedrige Konzentration), 1 mM (APC mittlere Konzentration) und 10 mM (APC Höchstkonzentration) über 24 Std. bei 37°C unter Standardzellkulturbedingungen inkubiert. Das Verhältnis von vitalen zu nonvitalen Zellen wurde mittels Auszählen unter dem Epifluoreszenz-mikroskop (Leica DMR) bestimmt.

## Beispiele

### Beispiel 1

### Monomolekulare Beschichtung einer Intraokularlinse mit einem Alkylphosphocholin

1.1. Theoretische Berechnungen

Für die Berechnung der Möglichkeit der "Modifikation der Oberfläche von Intraokularlinsen mit Alkylphosphocholinen, insbesondere Erucylphosphocholin (ErPC) und Erucylphosphohomocholin (Erufosin; $ErPC_3$), unter der Annahme der Ausbildung eines monomolekularen Oberflächenfilmes" gilt Folgendes:

Als Gesamtoberfläche der Intraokularlinse (d = 4 mm; r = 2 mm) wird eine Kugeloberfläche (S) zugrunde gelegt:

$$s = 4 * \pi * r^2$$
$$= 4 * 3.14 * 2.$$
$$= 50\ mm^2$$
$$= 50 * 10^{-6}\ [m^2]\ \text{(Gesamtoberfläche der IOL)}$$

Wenn man die Oberflächenbeanspruchung der Alkylphosphocholine an der Grenzfläche Wasser-Luft (Langmuir-Trog) heranzieht, so ergibt sich ein Wert von etwa 25 Å$^2$ / Molekül = 25 * $10^{-20}$ [m$^2$].

Wenn die Gesamtoberfläche der Linse nun durch die Oberflächenbeanspruchung eines Einzelmoleküls teilen, ergibt sich die Anzahl der Moleküle /Z), die sich in die Linsengrenzfläche einlagern können:

$$Z = \frac{50 * 10^{-6}\ [m^2]}{25 * 10^{-20}\ [m^2]}$$

$$Z = 2 * 10^{14}$$

Für Erufosin mit MG 503.75 gilt:

1 Mol = 504 g = 6 * $10^{23}$ Moleküle

Auf einer Linse befinden sich 2 * $10^{14}$ Moleküle. Damit ergibt sich für Erufosin:

$$1\ \text{Molekül} = \frac{504}{6 * 10^{23}}\ [g] = 84 * 10^{-23}\ [g]$$

Das Gewicht eines Einzelmoleküls Erufosin ist damit: 84 * $10^{-23}$ [g].

Auf der Linse befinden sich

$$2 * 10^{14}\ \text{Moleküle} = 2 * 10^{14} * 84 * 10^{-23}$$
$$= 1680 * 10^{-10}\ [g]\ \text{(durch MG = 504: Umwandlung g in mol)}$$
$$\triangleq 3 * 10^{-10}\ [mol]$$
$$= 0.3\ [nmol] = 300\ [pmol]$$

Diese Überlegungen zeigen, dass die in eine Linsenoberfläche eingelagerten $ErPC_3$-Moleküle ausreichen, um mit Massenspektroskopie die Menge an $ErPC_3$ quantitativ zu erfassen.

1.2. Experimentelle Beschichtung von Intraokularlinsen

10 Intraokularlinsen wurden jeweils separat in einem Reagenzglas mit 200 μl hochkonzentrierter Erufosin-Stammlösung (10mM $ErPC_3$ in Methanol oder Wasser) inkubiert. Die Linse wurde anschließend in ein neues Reagenzglas transferiert und getrocknet. Zur Extraktion des auf der Linse anhaftenden Erufosin wurde die Linse mit Methanol/Acetonitril (im Verhältnis 9:1) gespült. Erufosin ist in diesem Lösungsmittelgemisch gut löslich und auf der Linse anhaftende Erufosinmoleküle werden bei diesem Extraktionsschritt abgelöst. Die Menge an Erufosin wird anschließend quantitativ mittels Massenspektrometrie bestimmt. Dabei wurde ein Menge an ErPC pro Linse zwischen 280 und 330 pmol gemessen. Die experimentell bestimmte Menge entspricht gerade der theoretisch berechneten Menge für eine aus einem monomolekularen Film aus Erufosin bestehende Beschichtung.

Auf gleiche Weise können auch andere medizinische Materialien mit Phosphocholinen beschichtet werden.

1.3. Stabilität der Beschichtung

10 Intraokularlinsen wurden, wie in Beispiel 1.2 beschrieben, mit Erufosin beschichtet. Die Linsen wurden anschließend jeweils separat in neue Reagenzgläser transferiert und vier Waschschritten mit physiologischer Kochsalzlösung unterzogen. In einem weiteren Extraktionsschritt unter Verwendung von Methanol/Acetonitril (Verhältnis 9:1) wurde das auf den Linsen anhaftende Erufosin abgelöst und massenspektroskopisch bestimmt.

Die bestimmte Menge an Erufosin pro Linse lag zwischen 280 und 330 pmol. Auch bei mehrmaligen Waschen bzw. Spülen mit physiologischer Kochsalzlösung wurde somit keine merkliche Ablösung der Erufosin-Beschichtung von den Linsen festgestellt.

**Beispiel 2**

**Einfluss Alkylphosphocholine auf humane Linsen Epithelzellen**

2.1 Zellkultur von humanen Linsenepithelzellen

Die humane Linsenepithelzelllinie HLE-B3 wurde in Eagle's-modifiziertem essentiellen Medium (MEM; Biochrom, Berlin, Deutschland), supplementiert mit FCS, 50 IU Penicillin/ml und 50 μg Streptomycin/ml bei 37 °C in einem Inkubator in einer 5 % Kohlendioxidatmosphäre, kultiviert. Das Medium wurde jeden dritten Tag gewechselt. Trypsin-EDTA wurde zum Subkultivieren von Zellen nach Konfluenz nach 5 bis 7 Tagen verwendet. Das zelluläre Wachstum wurde täglich unter einem Leica PhasenkontrastMikroskop beobachtet.

Um die Proliferationscharakteristiken zu bestimmen, wurde das Wachstum durch Zellzählung unter Verwendung einer Neubauer-Kammer und eines automatisierten Zellzählers (CASY 1, Innovation, Karlsruhe, Deutschland) zu verschiedenen Zeitpunkten gemessen. Die maximale Proliferation wurde nach 72 Stunden gemessen.

2.2 Alkylphosphocholine

Es wurden die Alkylphosphocholine Erucyl-Phosphocholin sowie Erucylhomo-Phosphocholin (Erucyl-(N,N,N-trimethyl)-propylammonium) eingesetzt.

Die Alkylphosphocholine wurden in PBS gelöst und bei 4 °C gelagert. Anhand einer Verdünnungsreihe in PBS wurden APC-Endkonzentrationen in gleichen Volumina von PBS erhalten. Als Kontrolle wurden die gleichen Volumina von PBS ohne Zugabe von APCs in allen Experimenten verwendet.

2.3 Zelllebensfähigkeits-Assay

Die Zelllebensfähigkeit von HLE-B3 Zellen wurde nach zwei verschiedenen Methoden bewertet: dem Trypanblau-Ausschlusstest und dem Lebend-Tot-Assay. Der Trypanblau-Ausschlusstest wurde wie folgt durchgeführt: Nach der Inkubationsperiode wurden Proben von HLE-B3 Zellen in einer Hämozytometerkammer durch das Trypanblau-Ausschlussverfahren gezählt und der Anteil an toten Zellen wurde berechnet.

Der Lebend-Tot-Assay, ein Zweifarbenfluoreszenz-Assay, wurde verwendet, um die Zelllebensfähigkeit zu quantifizieren. Kerne von nicht lebensfähigen Zellen erscheinen rot aufgrund Färbung durch den Membran-impermeablen Farbstoff Propidiumiodid (Sigma-Aldrich), wohingegen die Kerne aller Zellen mit den Membran-permeablen Farbsoff Hoechst 33342 (Intergen, Purchase, NY) gefärbt wurden. Nahezu konfluente Kulturen von HLE-B3 Zellen, gezüchtet auf vier Kammern, wurden mit drei verschiedenen Konzentrationen von APCs für 24 Stunden behandelt. Um die Zelllebensfähigkeit zu bewerten, wurden die Zellen mit PBS gewaschen und mit 2,0 μg/ml Propidiumiodid und 1,0 μg/ml Hoechst 33342 für 20 Minuten bei 37 °C inkubiert. Anschließend wurden die Zellen mit einem Epifluoreszenz-Mikroskop

(Leica DMR, Bensheim, Deutschland) analysiert. Repräsentative Flächen wurden dann digital fotodokumentiert (Leica Image Capturer, Bensheim, Deutschland). Die markierten Kerne wurden in Fluoreszenz-Fotomikrographien gezählt. Tote Zellen wurden als Prozentsatz der Gesamtkerne in dem Feld angegeben. Die Daten basieren auf Zählungen in drei Experimenten, jeweils in duplikaten Vertiefungen durchgeführt, mit vier dokumentierten repräsentativen Feldern pro Vertiefung.

Die eingesetzten APC Konzentrationen betrugen 0,01 mM, 0,1 mM sowie 1 mM. Dieses Intervall wurde ausgewählt, um die abgeschätzte $IC_{50}$ Konzentration zu überspannen und um die Zelllebensfähigkeit bei potentiell effektiven Konzentrationen hinsichtlich Inhibition von Proliferation, Anhaftung und Migration in vitro zu bestimmen.

Für keine der analysierten Konzentrationen konnten morphologische Zelländerungen im Phasenkontrastmikroskop beobachtet werden. Die Zytotoxizität wie durch den Trypanblau-Ausschlusstest und den Lebend-Tot-Assay war nicht unterschiedlich bei APC-behandelten und den Kontrollzellen (siehe Figur 2).

2.4 Zellproliferationsassay

Der Tetrazolium Farbstoff-Reduktionsassay (MTT, 3-[4,5 Dimethyl-thiazol-2yl]-2,5-diphenyltetrazoliumbromid) wurde verwendet, um das Überleben der Zellen zu bestimmen. HLE-B3 Zellen (150 µl/Vertiefung bei einer Dichte von 5 x $10^4$ Zellen/Vertiefung) wurden in 96-"well"-Platten eingesetzt und mit verschiedenen Konzentrationen von APCs über 24 Stunden behandelt. Die unterschiedlichen Konzentrationen von 0,01 mM, 01 mM und 1 mM überspannten die 50 % Inhibitionskonzentration ($IC_{50}$), wie durch vorbereitende Assays bestimmt. Der $IC_{50}$ ist definiert als die Konzentration eines Wirkstoffes, die zu einer 50 % Reduktion der Zellzahl bei nicht-toxischen Konzentrationen führt.

Der MTT-Test wurde, wie bei Mosmann beschrieben, mit einigen Modifikationen durchgeführt. Nach Entfernung des Mediums wurden die Zellen mit PBS gewaschen und die MTT-Lösung wurde zugegeben. Die Zellen wurden bei 37 °C für 30 Minuten inkubiert. Nach drei Waschschritten mit PBS (pH 7,4) wurden die unlöslichen Formazan-Kristalle in Dimethylsulfoxid gelöst. Die optische Dichte in den Vertiefungen wurde unter Verwendung eines Mikroplattenauslesegerätes bei 550 nm (Molecular Probes, Garching, Deutschland) bestimmt. Die Ergebnisse aus den "wells" wurden als der mittlere Prozentanteil der Kontrollproliferation angegeben. Die Experimente wurden dreifach durchgeführt und drei Mal wiederholt. HLE-B3 Zellen der gleichen Passage, welche nur mit PBS inkubiert wurden, dienten als Kontrolle.

Die mit Alkylphosphocholinen beschichteten Intraokularlinsen werden in ein Zellkulturvolumen von 500 µl eingebracht, das dem Volumen des humanen Linsenkapselsackes, in den die Intraokularlinsen bei der Kataraktoperation am Menschen implantiert werden, entspricht. Außerdem werden die beschichteten Intraokularlinsen in vitro in eine Zellkultur mit bereits proliferierenden humanen Linsenepithelzellen eingebracht. Dies sind im Vergleich zu der in vivo Situation erschwerte Bedingungen, da es sich um bereits aktivierte Zellen handelt.

Die HLE-B3 Proliferation wurde signifikant nach einer einzelnen Behandlung der Zellen mit APCs, die dem Kulturmedium in Gegenwart von Serum zugegeben wurden, reduziert. Die folgenden Konzentrationen waren geeignet, Proliferation zu inhibieren: 0,01 mM ($p = 0{,}0048$); 0,1 mM ($p < 0{,}001$) und 1 mM ($p < 0{,}001$) (siehe Figur 3). Der beobachtete Effekt war konzentrationsabhängig und die $IC_{50}$ Konzentration wurde nahe 0,1 mM bestimmt.

2.5 Zellanhaftungsassay

96-well Platten (Nunc, Wiesbaden, Deutschland) wurden mit 70 µl/Vertiefung Fibronectin (50 µg/mL in PBS [pH 7,4]; Sigma-Aldrich) für 16 Stunden bei 4 °C beschichtet. Unspezifische Bindung wurde durch 2 mg/mL Ovalbumin (Sigma-Aldrich) in PBS für eine Stunde bei 37 °C blockiert. HLE-B3 Zellen wurden in 96-well Platten (Nunc) mit einer Dichte von 1 x $10^5$ Zellen/Vertiefung in 1 ml MEM, 20 % FCS eingesetzt. APCs in drei verschiedenen Konzentrationen (0,01 mM, 0,1 mM sowie 1 mM) wurden zugegeben. Nach vier Stunden wurden die Zellen dreimal sorgfältig gewaschen unter Verwendung einer automatisierten Plattenwaschvorrichtung (Molecular Devices, Garching, Deutschland).

Mit dem Tetrazoliumfarbstoff-Reduktionsassay (MTT) (Sigma-Aldrich), wurde die Zahl von nach dem Waschschritt anhaftenden Zellen, wie oben beschrieben, bestimmt. Die Zahl der anhaftenden lebensfähigen Zellen korrelierte mit der Absorbanz (optischen Dichte OD), gemessen durch den MTT-Test, bei 550 nm. Die Ergebnisse von den Vertiefungen wurden als mittlerer Prozentanteil der Kontrolle ausgedrückt (Kontroll-OD bei 550 nm zeichnet als 100 %).

Nach einer einzigen Behandlung von HLE-B3 Zellen waren APCs in der Lage, eine signifikante Inhibition von Zellanhaftung in Dosis-abhängiger Weise zu induzieren (siehe Figur 4). Nahe der $IC_{50}$ Konzentration, wie zuvor durch den MTT-Test bestimmt, reduzierten APCs Zellanhaftung sehr effektiv auf 66,5 % ($p < 0{,}001$ bei 0,1 mM), verglichen mit Kontrollen. Die maximale Inhibition von Zellanhaftung wurde bei einer APC-Konzentration von 1 mM (54,1 %) erreicht.

2.6 Zellmigrationsassay

Migration wurde mittels einer Modifikation der Boydens-Kammer-Methode unter Verwendung von Mikrochemotaxiskammern (Neuroprobe, Gaithersburg, MD, USA) und Polycarbonatfiltern (Nucleoprobe, Karlsruhe, Deutschland)

mit einer Porengröße von 8,0 μm bestimmt. Ein Fibronectinbeschichteter Filter wurde oben auf die untere Hälfte der Kammer angeordnet, die 180 μl MEM mit epidermalen Wachstumsfaktor (EGF-BB, PeptroTech, London) in einer Konzentration von 20 ng/ml enthielt. Die obere Hälfte der Kammer wurde mit einer Suspension von HLE-B3 Zellen bei einer Dichte von 2 x $10^5$ Zellen/ml in MEM und 1 % in FCS (500 μl) gefüllt. APCs wurden in zwei unterschiedlichen Konzentrationen, nämlich 0,1 mM und 1 mM, zugegeben. Die Zellen wurden für sechs Stunden bei 37 °C in 5 % $CO_2$ inkubiert. Um Zellen zu eliminieren, die nicht durch den Filter migriert waren, wurde die obere Seite des Filters mit einer Baumwollspitze abgeschabt. Dann wurde der Filter entfernt und in Methanol fixiert und anschließend mit Hematoxilyn und Eosin gefärbt. Die Zellzahl in fünf zufällig ausgewählten Bereichen wurde bei einer 200-fachen Vergrößerung unter Verwendung eines Phasenkontrastmikroskops (Leica Microsystems GmbH, Wetzlar, Deutschland) bestimmt. HLE-B3 Zellen der gleichen Passage, inkubiert mit gleichem Volumen an PBS ohne die Zugabe von APCs, dienten als Kontrolle.

Die HLE-B3 Migration wurde durch APCs effektiv inhibiert (siehe Figur 5).

Nahe an ihrer $IC_{50}$ Konzentration waren APCs in der Lage, eine Reduktion der Zellmigration auf 43,1 % (0,1 mM) der Zellen, verglichen mit den Kontrollen, zu induzieren. Eine APC Konzentration von 1 mM war in der Lage, eine maximale Migrationsinhibition von 95 % zu induzieren.

**Beispiel 3**

**Wirksamkeit einer mit APCs beschichteten Intraokularlinse zur Prophylaxe von Nachstar**

Intraokularlinsen werden, wie in Beispiel 1 erklärt, mit APC beschichtet und bei Raumtemperatur steril aufbewahrt. Humane Linsenepithelzellen werden wie unter Beispiel 2.1 in Petrischalen unter Standardzellkulturbedingungen kultiviert. Sobald eine Semikonfluenz der Zellen auf der jeweiligen Platte erreicht ist, wird eine mit APC beschichtete Intraokularlinse in das Zentrum der Platte gelegt. Als Kontrolle dienen nicht mit APC behandelte Intraokularlinsen. Nach 3, 5 und 7 Tagen werden die Linsen entfernt und die Zellzahl der verbliebenen Zellen in den Zellkulturschalen, wie unter 2.1 beschrieben, mittels Zellzähler und mittels des MTT-Tests bestimmt. Es zeigte sich eine deutlich reduzierte Anzahl von Linsenepithelzellen in den Zellkulturschalen, in die eine mit APC beschichtete Intraokularlinse eingebracht wurde.

In Figur 6 (Säulendiagramm zur Inhibition der humanen Linsenepithelproliferation durch Oley-Phosphocholin-beschichete Intraokularlinsen) ist gezeigt, dass Intraokularlinsen aus verschiedenen Materialien (hydrophobe Acrylate, hydrophile Acrylate und Silikone), mit unterschiedlichen Haptik-Designs und Optikdurchmessern mit Alkylphosphocholinen beschichtet (OlPC) das Zellwachstum von bereits proliferierenden Linsenepithelzellen hemmen können.

Zur Bestimmung der Zellzahl wurde eine Tetrazoliumreduktionsassays [MTT (3-[4,5-dimethylthiazol- 2-yl] -2,5-diphenyltetrazolium bromide]; (Sigma-Aldrich) durchgeführt. Die Intraokularlinse wurde entnommen, die Zellen wurden mit PBS gewaschen und mit dem MTT Reagenz für 1 Stunde bei 37°C inkubiert. Nur vitale Zellen verstoffwechseln die Lösung. Anschließend wurde die Zellmembran mittels DMSO (Dimethylsulfoxid) gelöst und die optische Dichte im ELISA-Reader bei 550 nm gemessen. Die optische Dichte korreliert hierbei mit der Anzahl der proliferierenden Zellen.

In Figur 7 (Säulendiagramm zur Inhibition der humanen Linsenepithelproliferation durch Erucyl-Phosphohomocholin-beschichete Intraokularlinsen) ist dies auch für Intraokularlinsen aus verschiedenen Materialien (hydrophobe Acrylate, hydrophile Acrylate und Silikone), mit unterschiedlichen Haptik-Designs und Optikdurchmessern, die mit $ErPC_3$ beschichtet wurden, gezeigt.

**Beispiel 4**

**Sicherheitsprofil der Substanzklasse der Alkylphosphocholine am Modell der isolierten perfundierten bovinen Netzhaut**

Die Sicherheit von Alkylphosphocholinen (APC) im ex vivo Modell der perfundierten bovinen Netzhaut wurde untersucht. Dazu wurden Präparationen der bovinen Netzhaut mit einer Sauerstoff-präequilibrierten Standardlösung perfundiert. Das Elektroretinogramm (ERG) wurde unter Verwendung von Ag/AgCl-Elektroden aufgenommen. Nachdem stabile b-Wellenamplituden aufgezeichnet wurden, wurde Alkylphosphocholin mit den Konzentrationen 0,25 μM, 2,5 μM und 25 μM der Nährlösung zugegeben. Um die Effekte von Alkylphosphocholinen auf die Photorezeptorfunktion zu erfassen, wurde eine Testreihe mit den gleichen Konzentrationen durchgeführt, bei der die Effekte von Alkylphosphocholin auf die a-Wellenamplitude beurteilt wurde. Hierzu wurde Aspartat in einer Konzentration von 1 μM der Nährlösung zugegeben, um stabile a-Wellenamplituden zu erhalten. Danach wurde Alkylphosphocholin mit den gleichen Konzentrationen der Nährlösung zugegeben. Die ERG-Amplituden wurden für 75 Minuten beobachtet.

Es wurden keine Reduktionen der a- und b-Wellenamplitude am Ende der Behandlung mit Alkylphosphocholin in den Testserien beobachtet. Es wurden keine Unterschiede zwischen den ERG-Amplituden vor und nach der Behandlung mit Alkylphosphocholin festgestellt.

**Beispiel 5**

**Beschichtung von Intraokularlinsen mit APCs**

In einer 6-well Platte wird well 1 mit 1,5 ml APC Stammlösung und well 3 mit einer 1,5 ml physiologischen Kochsalzlösung gefüllt. Eine Intraokularlinse (IOL) wird in well 1 in die APC Stammlösung (10 mM APC in physiologischer Kochsalzlösung) eingebracht und über Nacht bei Raumtemperatur inkubiert. Anschließend wird die Intraokularlinse entnommen und in das leere well 2 gelegt und 3 Stunden bei 4 °C inkubiert. Danach wird die Intraokularlinse in well 3 (physiologische Kochsalzlösung) transferiert und 1 Stunde bei 37 °C inkubiert. Nach Entnahme der Intraokularlinse wird die 6-well Beschichtungsplatte mit Folie umwickelt und bei 4 °C aufbewahrt.

Die mit APC beschichtete Intraokularlinse (APC-IOL) wird auf proliferierende HLE-B3 Zellen (6-well Platte, ca. 60 % Konfluenz) gelegt (Volumen 1,5 ml MEM/5% FCS). Als Kontrolle diente eine Intraokularlinse gleichen Typs ohne APC-Beschichtung.

Die Zellen wurde alle zwei Tage auf Wachstum/Überwachsen der IOL bzw. Migration auf die IOL hin untersucht und fotodokumentiert.

Nach 10 bis 14 Tagen werden die IOLs entnommen und die Zellen abgelöst und die Zellzahl bestimmt und verglichen. Die Bestimmung der Zellzahl erfolgte in einer Neubauer Zählkammer bzw. in einem Casy Cell Counter).

**Beispiel 6**

**Verhinderung einer Nachstar-Bildung auf mit Alkylphosphocholinen beschichteten Intraokularlinsen**

In diesem Beispiel wurde die Fähigkeit von mit APC beschichteten Intraokularlinsen untersucht, Proliferation von humanen Linsenepithelzellen zu inhibieren. Dazu wurden Intraokularlinsen (IOLs) unterschiedlichen Designs (dreistückige und einstückige IOLs) in eine Vorratslösung von APC eingebracht mit PBS gewaschen und über Nacht getrocknet. Unbeschichtete IOLs mit den gleichen Designs dienten als Kontrolle. Die IOLs wurden jeweils in eine well einer 24-well Platte gegeben, welche proliferierende humane Linsenepithelialzellen (HLE-B3) enthielten, und unter Standard-Zellkulturbedingungen für drei Tage kultiviert. Dann wurde der MTT-Test durchgeführt und die Zellzahl pro well berechnet. An Tag 2 und 3 wurden die Zellen neben und unter der Intraokularlinse photodokumentiert (Figur 8). Es zeigt sich, dass die Alkylphosphocholin-beschichtete Intraokularlinse, wenn An Tag 2 und 3 wurden die Zellen neben und unter der Intraokularlinse photodokumentiert (Abbildung 8). Es zeigt sich, dass die Alkylphosphocholin-beschichtete Intraokularlinse, wenn sie in Kultur eingebracht wird, zu einer Inhibition der zellulären Ausbreitung und des Wachstums der Zellen sowohl neben als auch unter der Intraokularlinse. Der Effekt verstärkt sich über die Zeit (Zunahme des Effektes von 48 auf 72 Std nach Einbringen der Alkylphosphocholin beschichteten Intraokularlinsen in die Zellkultur).

Die Untersuchungen ergaben, dass die Proliferation von humanen Linsenepithelialzellen durch mit APC beschichteten Intraokularlinsen inhibiert wurde. Die Ergebnisse, angegeben in Prozent bezogen auf die Kontrollen, waren wie folgt:

39 % $\pm$ 25 für einstückige IOLs
und
79 % $\pm$ 10 für dreistückige IOLs.

Dies zeigte, dass die Zellproliferation auf einstückigen IOLs effektiver inhibiert wurde als die Zellproliferation auf dreistückigen IOLs.

Es zeigt sich, dass insbesondere einstückige Intraokularlinsen von der Alkylphosphocholin-Beschichtung profitieren.

Die Versuche zeigten, dass APCs geeignete Beschichtungsmittel für Intraokularlinsen sind, mit denen die Proliferation von humanen Linsenepithelzellen signifikant inhibiert werden kann. Solche Linsen können somit zur pharmakologischen Prophylaxe gegen eine Nachstarbildung (posterior capsule opacification) eingesetzt werden.

**Patentansprüche**

1. Medizinisches Material,
**dadurch gekennzeichnet,**
**dass** es eine Alkylphosphocholin-Verbindung der Formel (I)

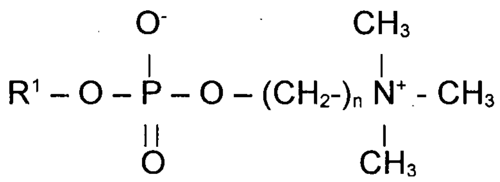

(I)

enthält, worin
$R^1$ ein $C_{16}$-$C_{24}$-Kohlenwasserstoffrest ist, welcher eine oder mehrere ungesättigte Bindungen enthalten kann, und
n eine ganze Zahl von 1 bis 5 darstellt.

2. Medizinisches Material nach Anspruch 1,
**dadurch gekennzeichnet,**
**dass** es mit einer Alkylphosphocholin-Verbindung beschichtet ist oder/und eine Alkylphosphocholin-Verbindung dem Grundmaterial beigemischt ist.

3. Medizinisches Material nach einem der vorhergehenden Ansprüche,
**dadurch gekennzeichnet,**
**dass** es wenigstens Teil eines ophthalmologischen Implantats oder/und dass es wenigstens Teil einer Intraokularlinse ist.

4. Medizinisches Material nach Anspruch 1 oder 2,
**dadurch gekennzeichnet,**
**dass** es wenigstens Teil eines medizinischen Implantats oder/und wenigstens Teil eines in der Diagnose, Therapie, Prophylaxe oder/und zur Verbesserung von unerwünschten Zuständen eingesetzten medizinischen Materials ist.

5. Medizinisches Material nach einem der Ansprüche 1 oder 2,
**dadurch gekennzeichnet,**
**dass** es wenigstens Teil eines Stents, einer Herzklappe, eines Verweilkatheters, einer Prothese, eines Implantats oder/und eines Nahtmaterials oder/und einer Kontaktlinse ist.

6. Medizinisches Material nach einem der vorhergehenden Ansprüche,
**dadurch gekennzeichnet,**
**dass** es ausgewählt ist aus hydrophoben Acrylaten, hydrophilen Acrylaten und Silikon.

7. Medizinisches Material nach einem der vorhergehenden Ansprüche,
**dadurch gekennzeichnet,**
**dass** die Alkylphosphocholin-Verbindung Erucylphosphocholin oder Erucylhomophosphocholin oder Oleylphosphocholin ist.

8. Medizinisches Material nach einem der vorhergehenden Ansprüche zur Verbesserung der Wundheilungsreaktion und/oder zur Verminderung von Fremdkörperreaktionen.

9. Medizinisches Material nach einem der vorhergehenden Ansprüche, wenigstens als Teil eines ophthalmologischen Implantats zur Prophylaxe oder/und Verhinderung von Nachstar.

10. Verwendung einer Alkylphosphocholin-Verbindung der Formel (I)

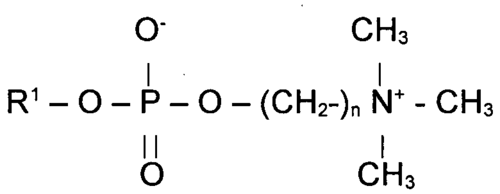

(I)

worin
$R^1$ ein $C_{16}$-$C_{24}$ Kohlenwasserstoffrest ist, welcher eine oder mehrere ungesättigte Bindungen enthalten kann, und
n eine ganze Zahl von 1 bis 5 darstellt
bei der Herstellung eines medizinischen Materials.

**11.** Verwendung nach Anspruch 10 bei der Herstellung eines ophthalmologischen Implantats zur Prophylaxe oder/und Verhinderung von Nachstar.

## Claims

**1.** A medical material, **characterised in that** it contains an alkylphosphocholine compound of formula (I)

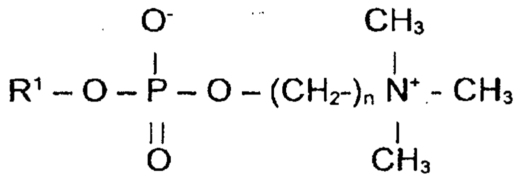

(I)

in which
$R^1$ is a $C_{16}$-$C_{24}$ hydrocarbon residue which may contain one or more unsaturated bonds, and
n represents an integer from 1 to 5.

**2.** A medical material according to claim 1, **characterised in that** it is coated with an alkylphosphocholine compound and/or an alkylphosphocholine compound is incorporated into the base material.

**3.** A medical material according to either one of the preceding claims, **characterised in that** it is at least part of an ophthalmological implant and/or **in that** it is at least part of an intraocular lens.

**4.** A medical material according to claim 1 or claim 2, **characterised in that** it is at least part of a medical implant and/or at least part of a medical material used in the diagnosis, treatment, prophylaxis and/or improvement of undesired conditions.

**5.** A medical material according to either of claim 1 or claim 2, **characterised in that** it is at least part of a stent, a heart valve, an indwelling catheter, a prosthesis, an implant and/or a suture material and/or a contact lens.

**6.** A medical material according to any one of the preceding claims, **characterised in that** it is selected from hydrophobic acrylates, hydrophilic acrylates and silicone.

**7.** A medical material according to any one of the preceding claims, **characterised in that** the alkylphosphocholine compound is erucylphosphocholine or erucylhomophosphocholine or oleylphosphocholine.

**8.** A medical material according to any one of the preceding claims for improving wound-healing response and/or for reducing foreign-body reactions.

**9.** A medical material according to any one of the preceding claims, at least as part of an ophthalmological implant for prophylaxis and/or prevention of posterior capsule opacity.

**10.** Use of an alkylphosphocholine compound of formula (I)

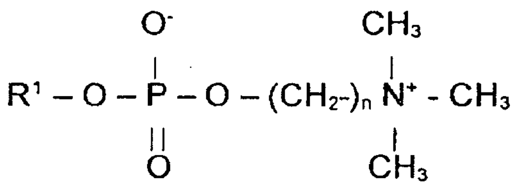

(I)

in which
$R^1$ is a $C_{16}$-$C_{24}$ hydrocarbon residue which may contain one or more unsaturated bonds, and
n represents an integer from 1 to 5
in the production of a medical material.

**11.** Use according to claim 10 in the production of an ophthalmological implant for prophylaxis and/or prevention of posterior capsule opacity.

## Revendications

**1.** Matériau médical, **caractérisé en ce qu'**il contient un composé alkylphosphocholine de formule (I)

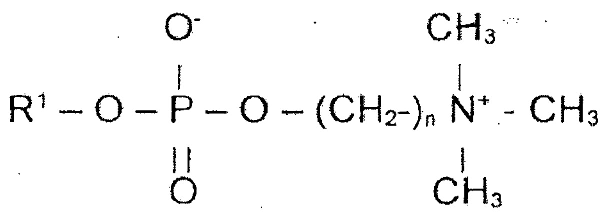

(I)

dans laquelle $R^1$ est un radical hydrocarboné en $C_{16}$-$C_{24}$, qui peut contenir une ou plusieurs liaisons insaturées, et n est un nombre entier de 1 à 5.

**2.** Matériau médical selon la revendication 1, **caractérisé en ce qu'**il est revêtu d'un composé alkylphosphocholine, et/ou qu'un composé alkylphosphocholine est mélangé au matériau de base.

**3.** Matériau médical selon l'une des revendications précédentes, **caractérisé en ce qu'**il est au moins une partie d'un implant ophtalmologique et/ou qu'il est au moins une partie d'une lentille intraoculaire.

**4.** Matériau médical selon la revendication 1 ou 2, **caractérisé en ce qu'**il est au moins une partie d'un implant médical et/ou au moins une partie d'un matériau médical utilisé en diagnostic, en thérapie, en prophylaxie et/ou pour l'amélioration d'états indésirables.

**5.** Matériau médical selon l'une des revendications 1 ou 2, **caractérisé en ce qu'**il est au moins une partie d'un stent, d'une valvule cardiaque, d'un cathéter à demeure, d'une prothèse, d'un implant et/ou d'un matériau de suture et/ou d'une lentille de contact.

**6.** Matériau médical selon l'une des revendications précédentes, **caractérisé en ce qu'**il est choisi parmi les acrylates hydrophobes, les acrylates hydrophiles et la silicone.

**7.** Matériau médical selon l'une des revendications précédentes, **caractérisé en ce que** le composé alkylphosphocholine est l'érucylphosphocholine ou l'érucylhomophosphocholine ou l'oléylphosphocholine.

**8.** Matériau médical selon l'une des revendications précédentes, pour l'amélioration de la réaction de cicatrisation et/ou pour diminuer les réactions de rejet des corps étrangers.

**9.** Matériau médical selon l'une des revendications précédentes, au moins en tant que partie d'un implant ophtalmologique pour la prophylaxie et/ou la prévention de la cataracte secondaire.

**10.** Utilisation d'un composé alkylphosphocholine de formule (I)

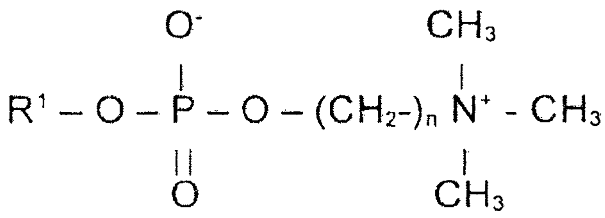

(I)

dans laquelle $R^1$ est un radical hydrocarboné en $C_{16}$-$C_{24}$, qui peut contenir une ou plusieurs liaisons insaturées, et n est un nombre entier de 1 à 5, pour la fabrication d'un matériau médical.

**11.** Utilisation selon la revendication 10 pour la fabrication d'un implant ophtalmologique pour la prophylaxie et/ou la prévention de la cataracte secondaire.

Figur 1

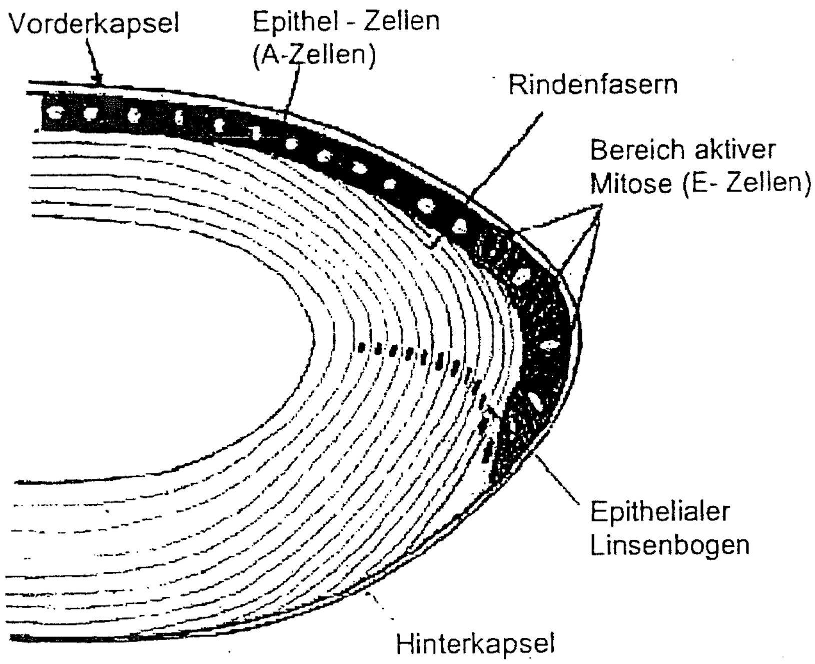
Vorderkapsel
Epithel - Zellen
(A-Zellen)
Rindenfasern
Bereich aktiver
Mitose (E- Zellen)
Epithelialer
Linsenbogen
Hinterkapsel

Figur 2

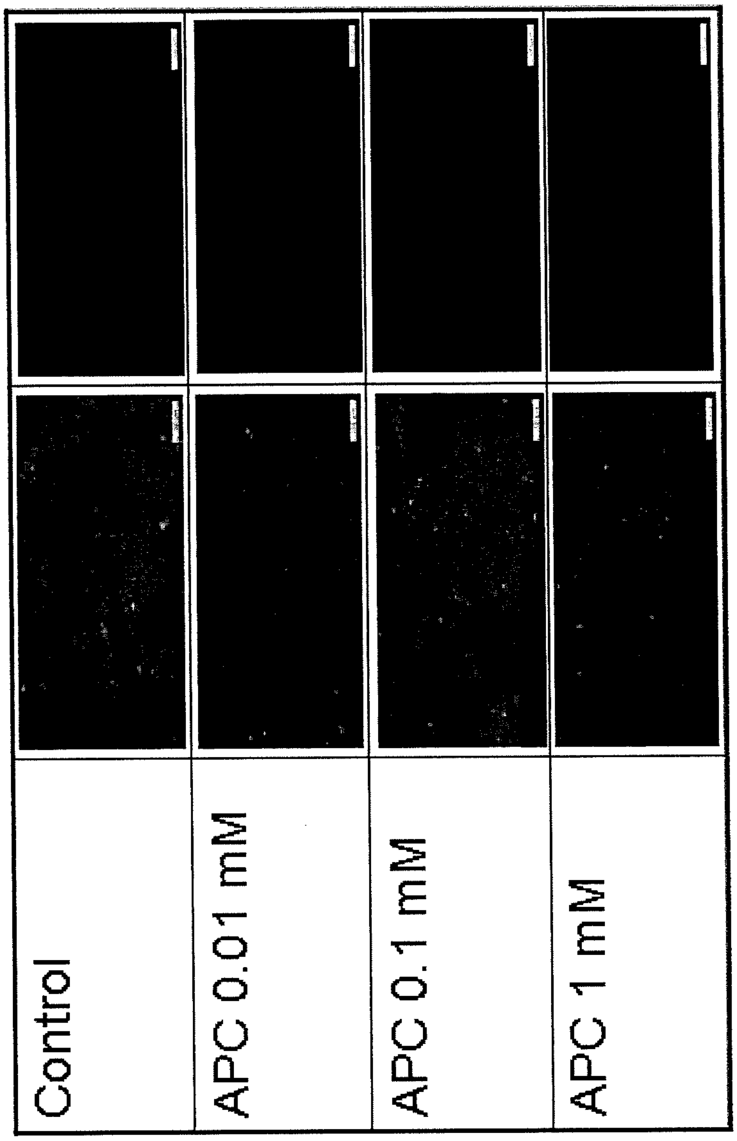

Figur 3

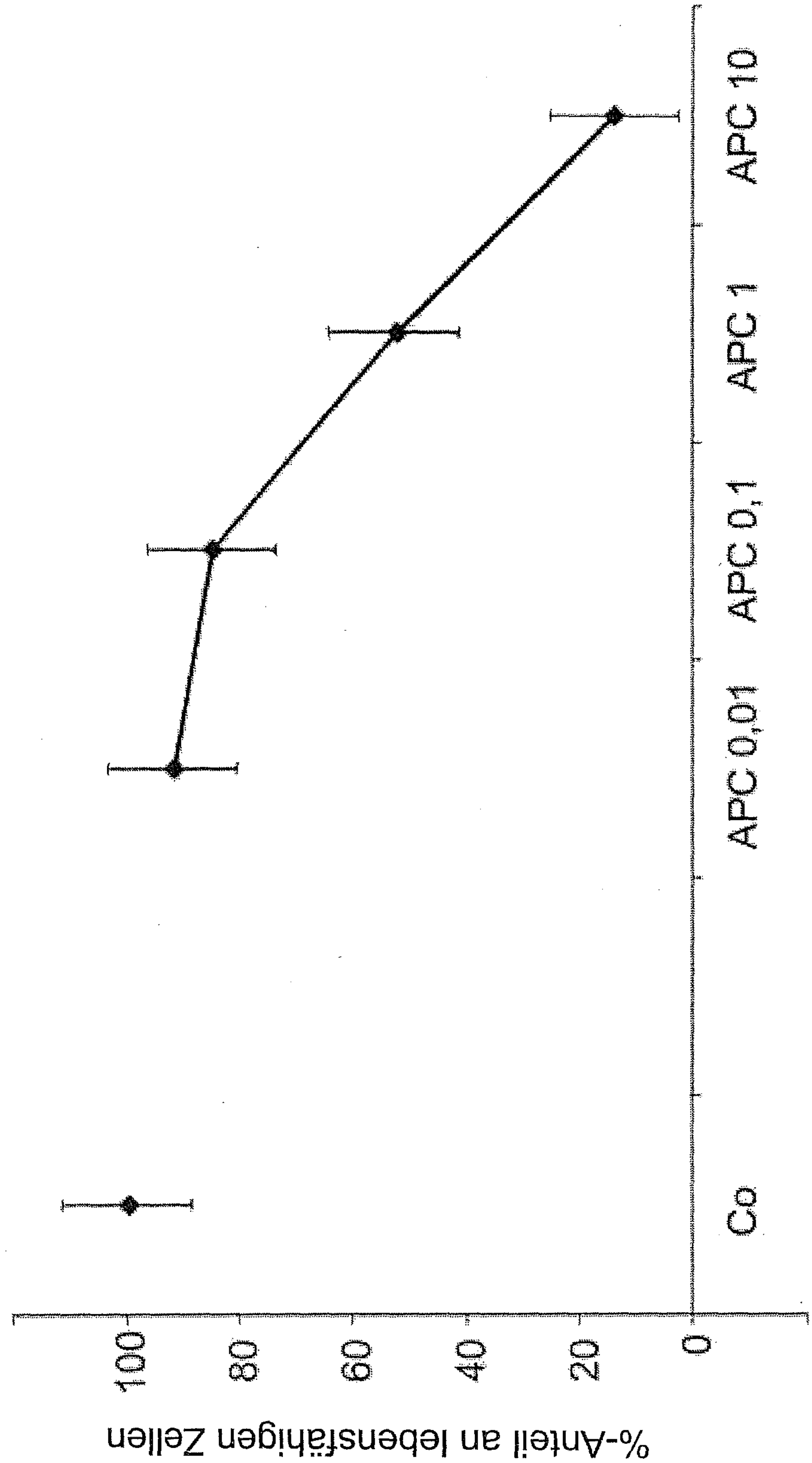
%-Anteil an lebensfähigen Zellen
100
80
60
40
20
0
Co
APC 0,01
APC 0,1
APC 1
APC 10

Figur 4

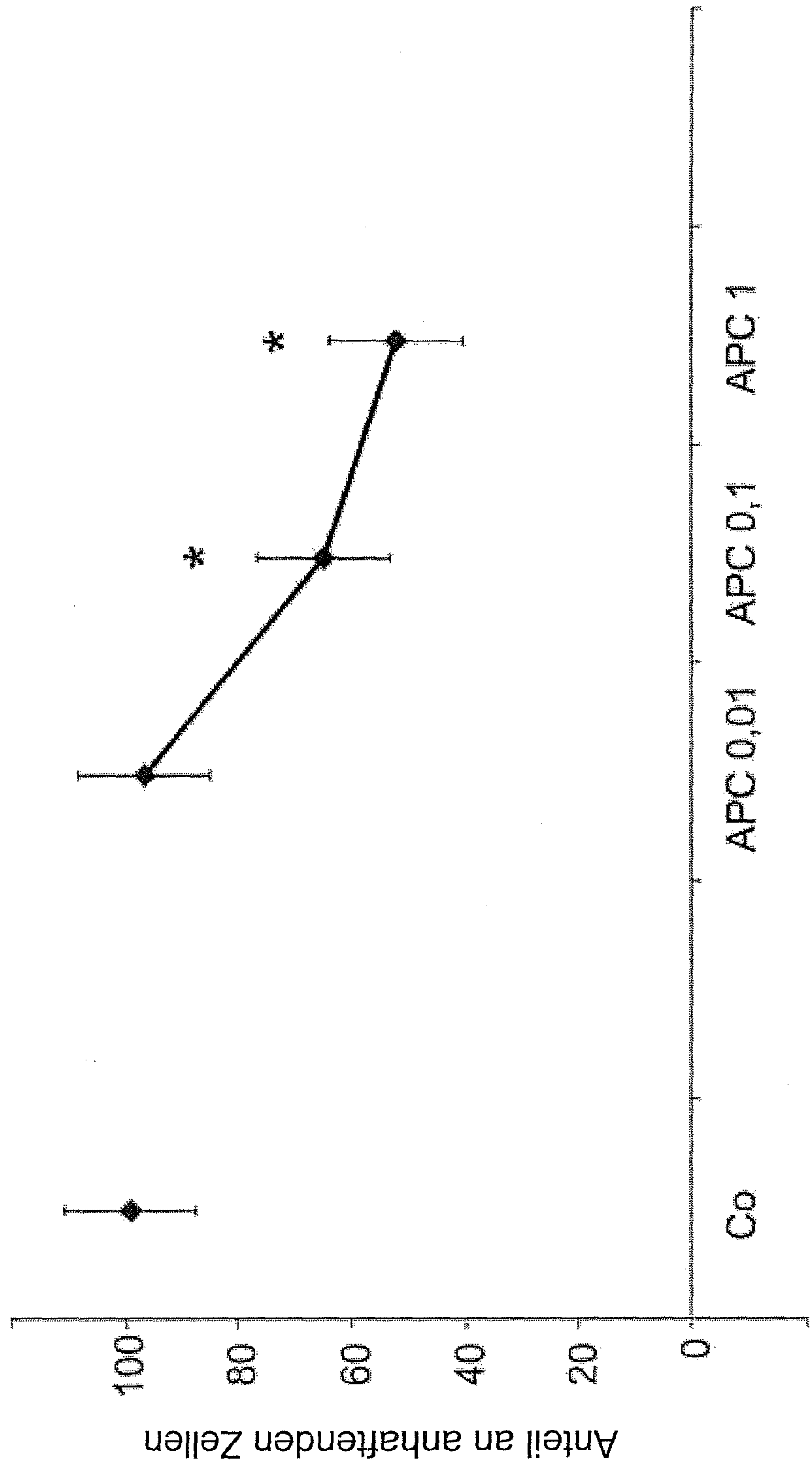
Anteil an anhaftenden Zellen
0
20
40
60
80
100
Co
APC 0,01
APC 0,1
APC 1
*
*

Figur 5

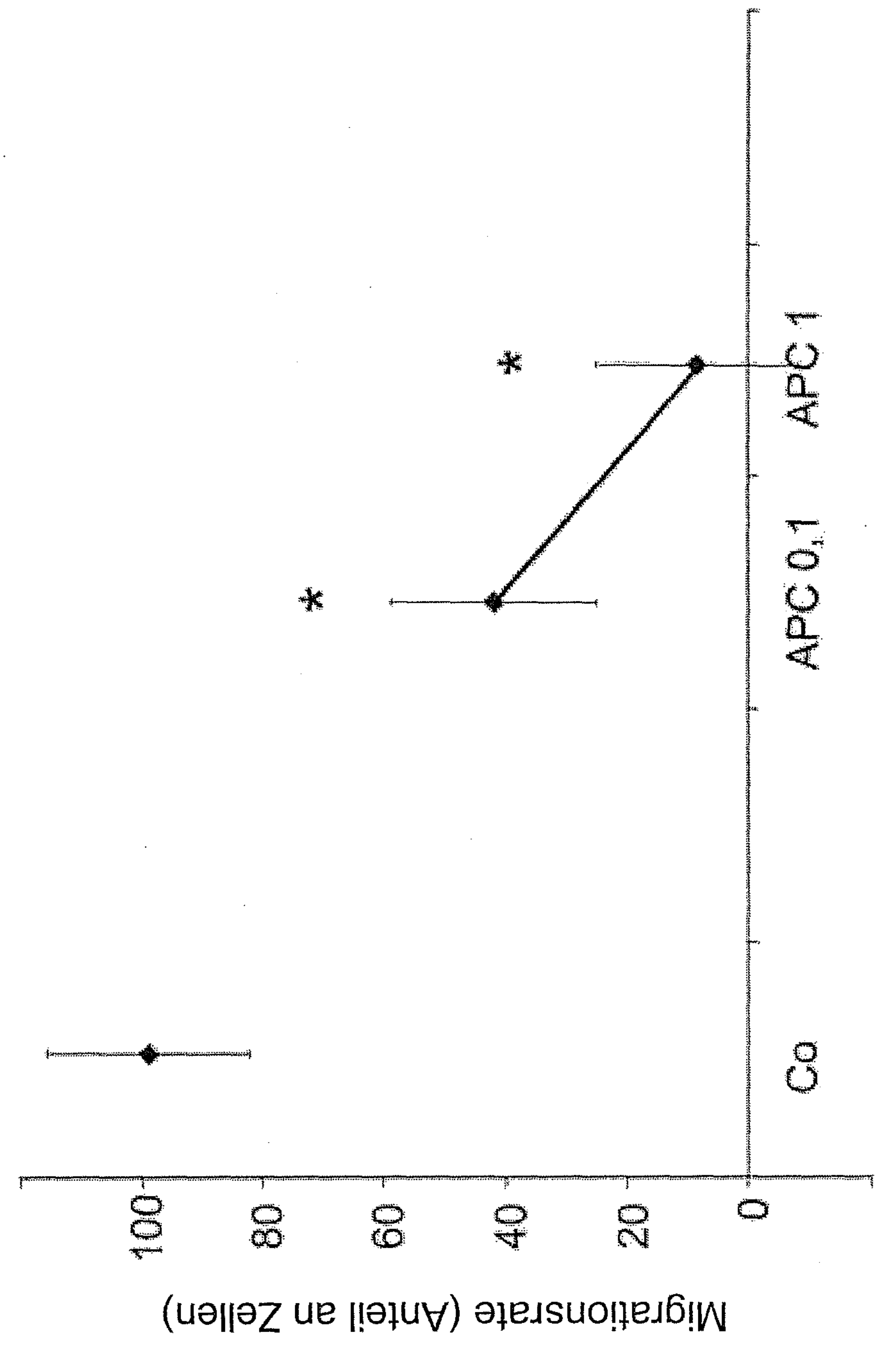
Migrationsrate (Anteil an Zellen)
100
80
60
40
20
0
Co
APC 0,1
APC 1
*
*

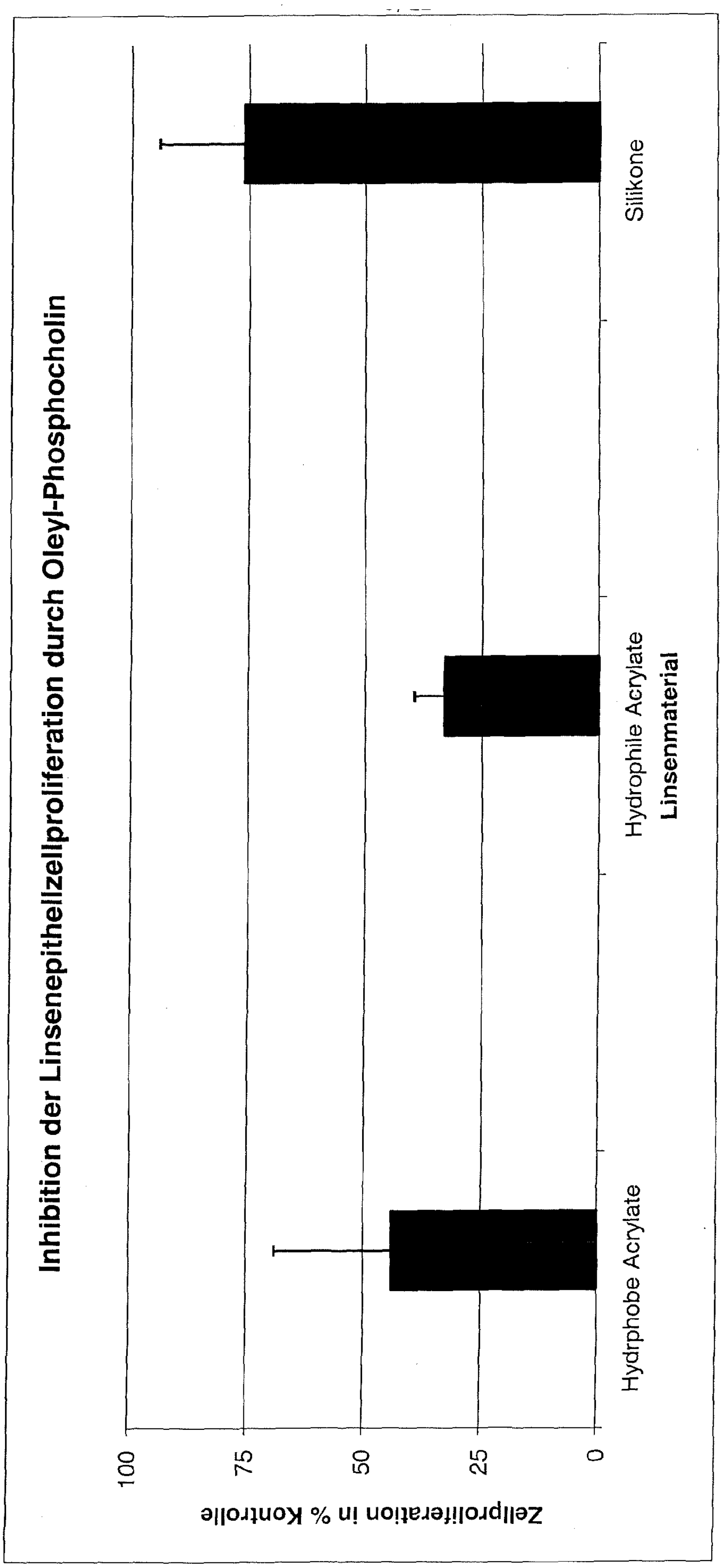
Inhibition der Linsenepithelzellproliferation durch Oleyl-Phosphocholin
Zellproliferation in % Kontrolle
100
75
50
25
0
Hydrphobe Acrylate
Hydrophile Acrylate
Silikone
Linsenmaterial

Figur 6

Figur 7

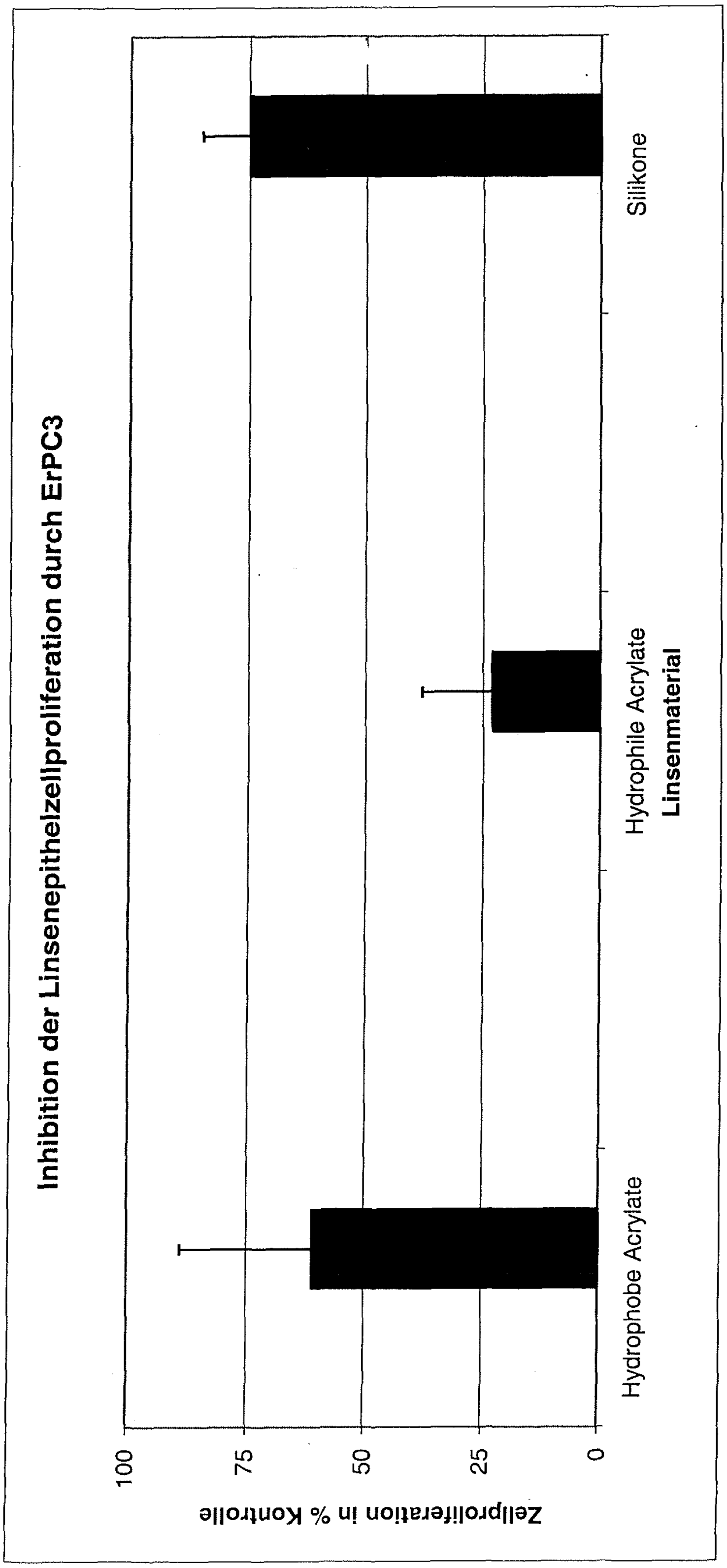
Inhibition der Linsenepithelzellproliferation durch ErPC3
Zellproliferation in % Kontrolle
100
75
50
25
0
Hydrophobe Acrylate
Hydrophile Acrylate
Silikone
Linsenmaterial

APC beschichtete Intraokularlinse nach 48 Std.

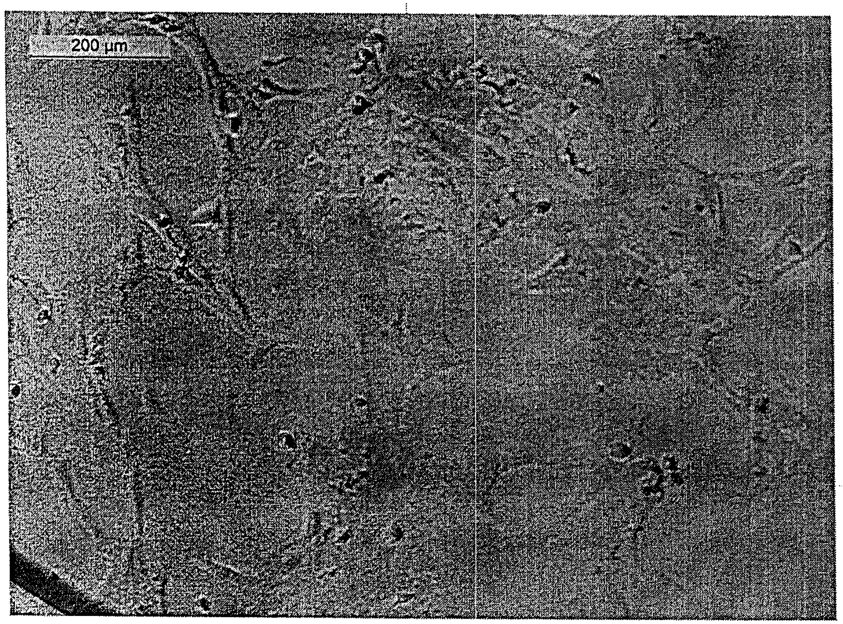


Zellen neben Kontroll-IOL

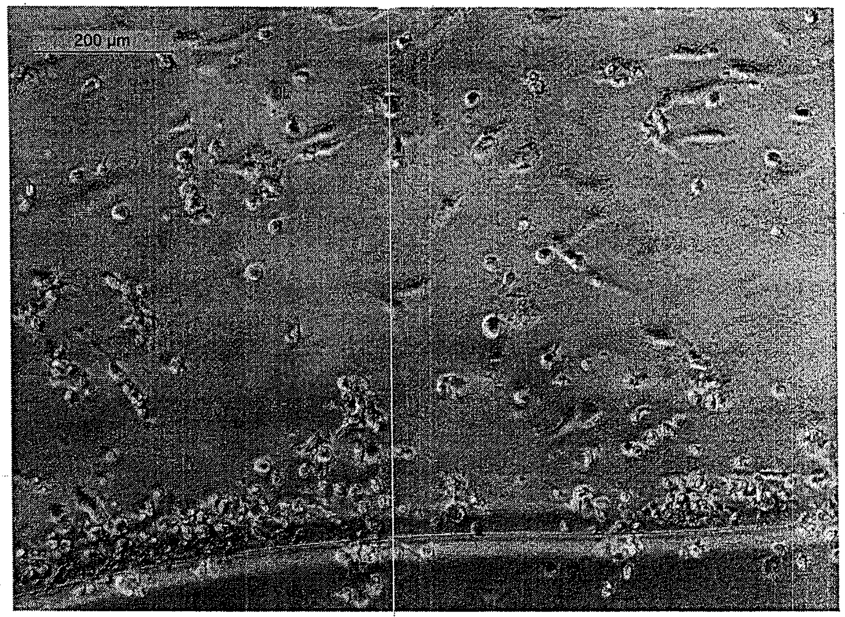


Zellen neben APC-IOL

Figur 8a

Figur 8b

APC beschichtete Intraokularlinse nach 48 Std

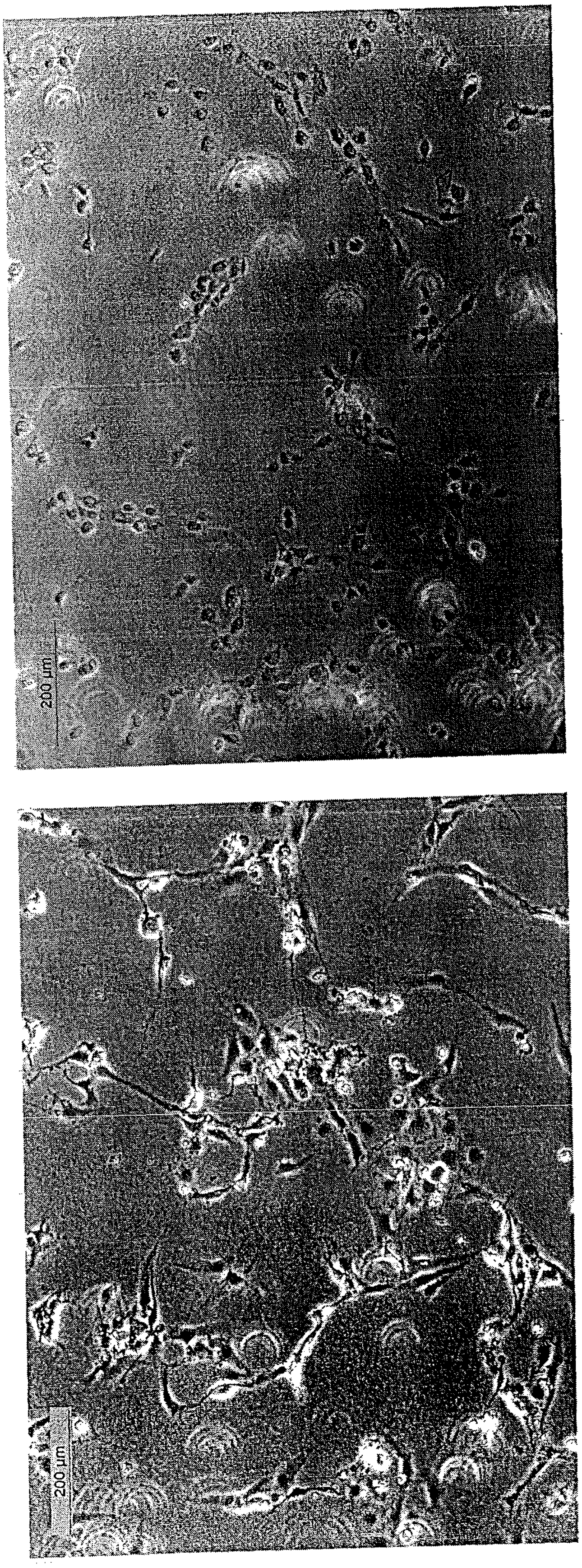


Zellen unter Kontroll-IOL

Zellen unter APC-IOL

Figur 8c

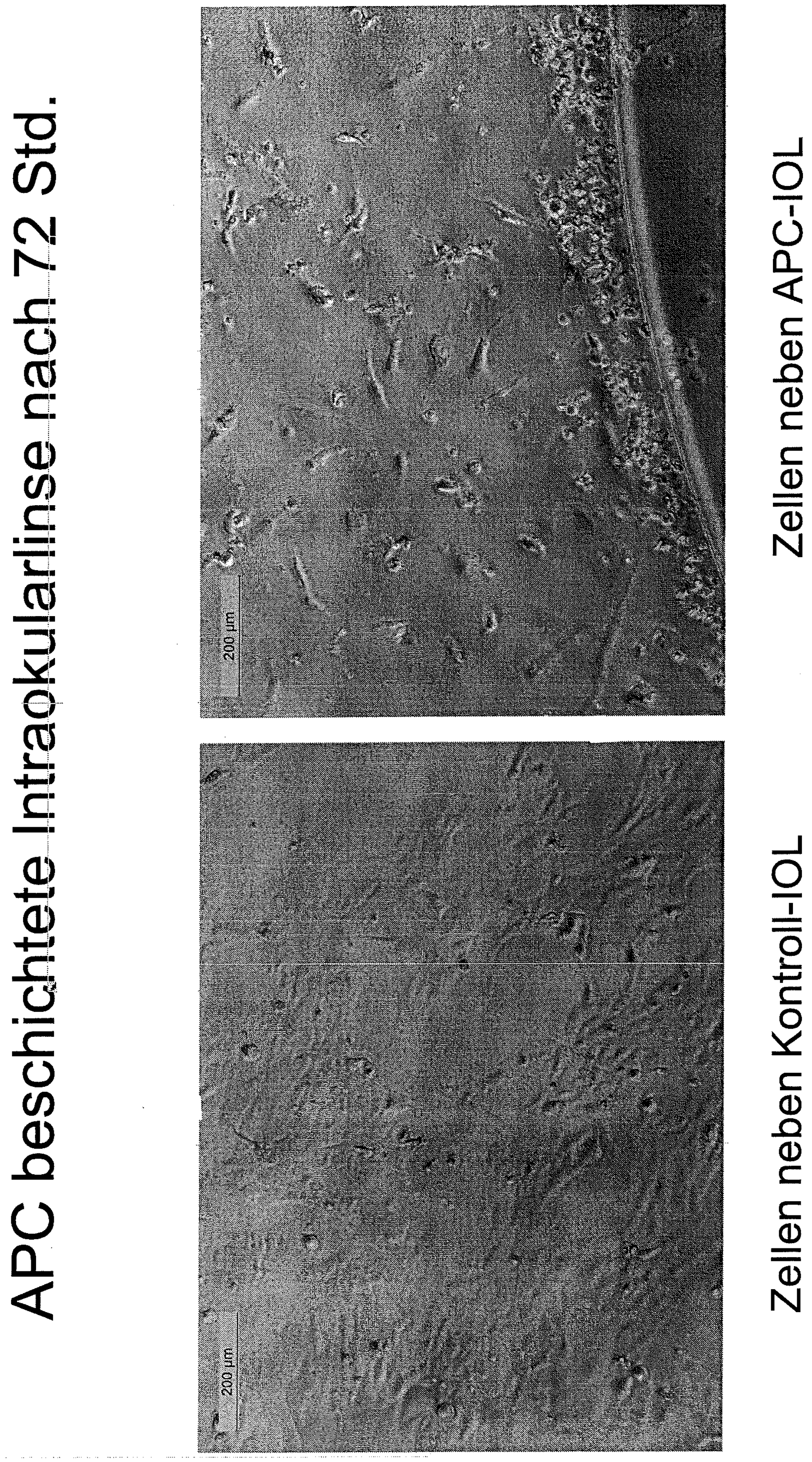
APC beschichtete Intraokularlinse nach 72 Std.
Zellen neben APC-IOL
Zellen neben Kontroll-IOL
200 µm
200 µm

Figur 8d

APC beschichtete Intraokularlinse nach 72 Std

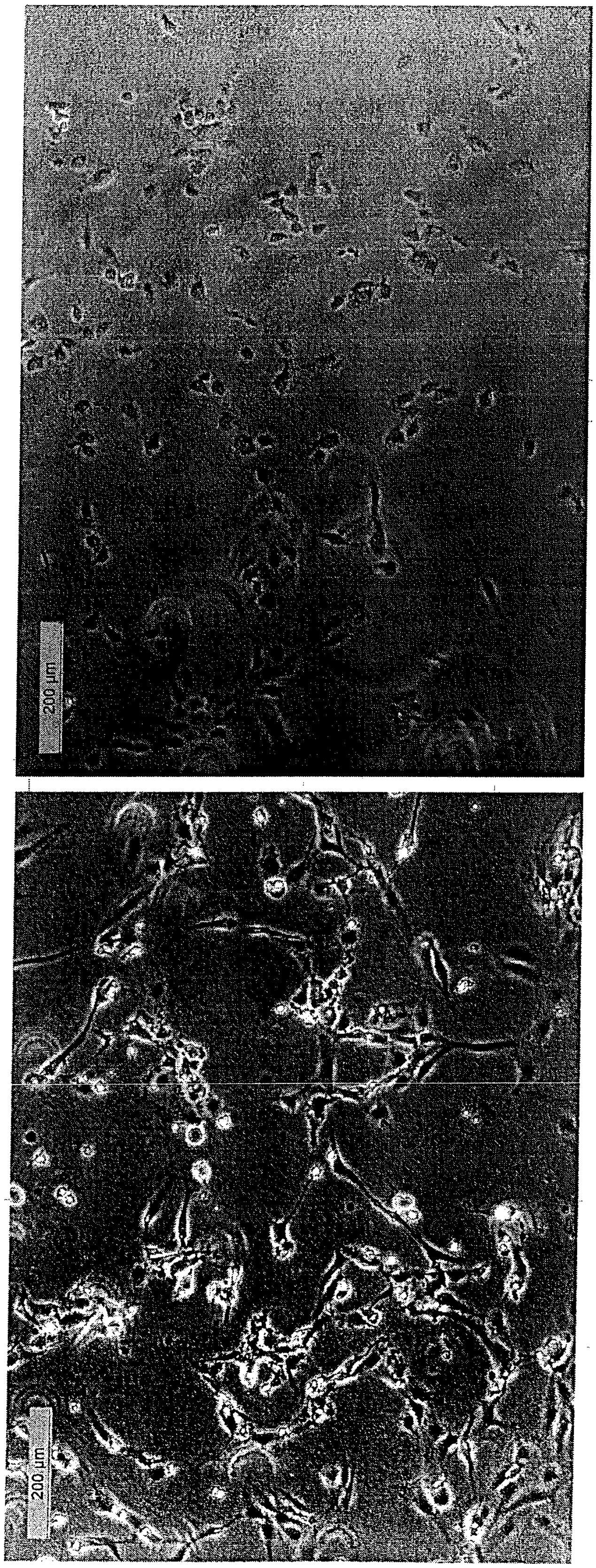


Zellen unter APC-IOL

Zellen unter Kontroll-IOL

Figur 9

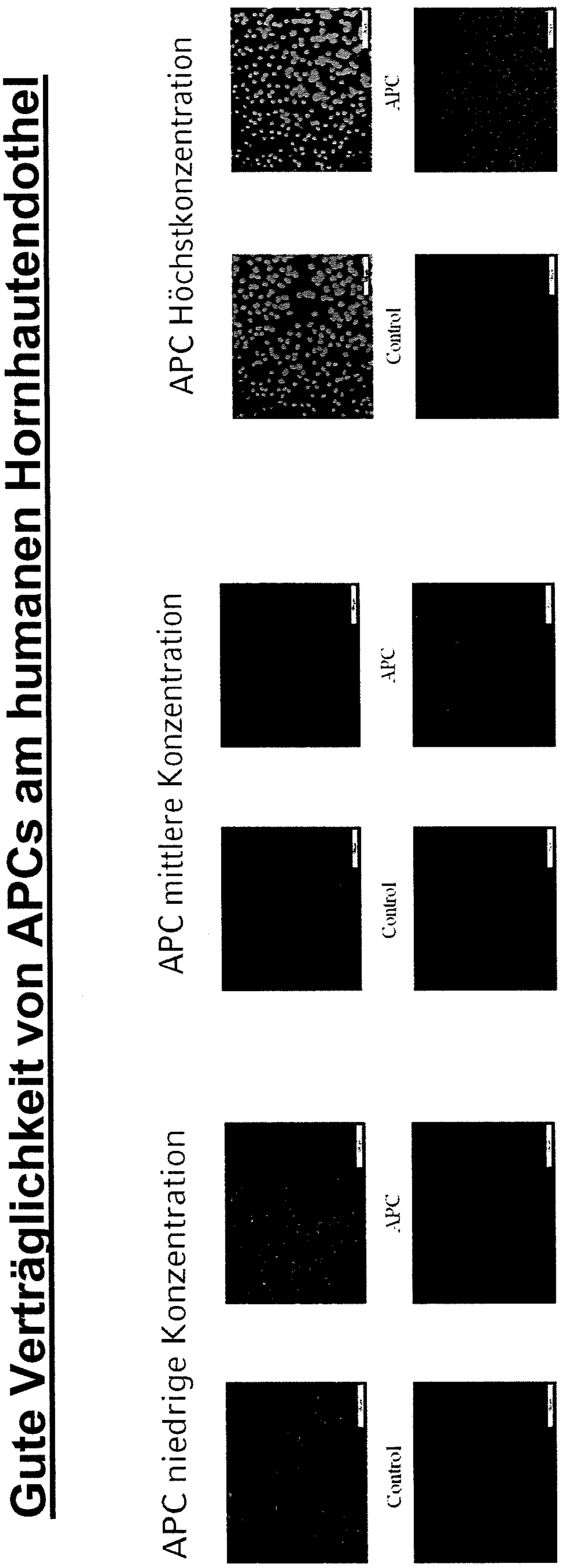
Gute Verträglichkeit von APCs am humanen Hornhautendothel
APC niedrige Konzentration
Control
APC
APC mittlere Konzentration
Control
APC
APC Höchstkonzentration
Control
APC

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5342621 A
- US 20030219909 A1
- US 20030219909 A
- US 20080090781 A1


### In der Beschreibung aufgeführte Nicht-Patentliteratur


- **SHIGETA et al.** *J. Cataract. Refract. Surg.,* Mai 2006, vol. 32, 859-864
- **KLEIN et al.** *Ophthalmology,* 1984, vol. 91, 1-9
- **SCHEIN et al.** *N Engl J Med,* 2000
- **KUGELBERG et al.** *J Cat Refract Surg,* 2005, vol. 31, 757-762
- **MASTROPASQUA et al.** *Acta Ophthalmol Scand,* 2007, vol. 85, 409-414
- **FINDL et al.** *Cochrane Database of Systematic Reviews,* 2007
- **CLEARY ; SPALTON.** *ESCRS Eurotimes,* 2008, vol. 115, 1308-1314
- **KOHNEN et al.** *Ophthalmology,* 2008
- **CHUNG et al.** *J Cataract Refract Surg,* 2000
- **NISHI et al.** *J Cataract Refract Surg,* 1999
- **RUIZ et al.** *Ophthalmic Res,* 1990
- **POWER et al.** *J Cataract Refract Surg,* 1994
- **KIM et al.** *Clin Exp Ophthalmol,* 2007
- **ABDELWAHAB et al.** *Eye,* 2008
- **MALOOF et al.** *Arch Ophthalmol,* 2005
- **RABSILBER et al.** *Br J Ophthalmol,* 2007
- **KANG et al.** *Eur J Ophthalmol,* 2008
- **NISHI et al.** *J. Cat. Refract. Surg.,* 1998
- **KOOPMANS et al.** *Invest Ophthalmol Vis Sci,* 2003
- **EIBL H et al.** *Cancer Treat Rev,* 1990
- **LEONARD et al.** *J Clin Oncol,* 2001
- **SUNDAR et al.** *N Engl J Med,* 2002
- **SCHÜTTAUF et al.** *Curr Eye Res,* 2005
- **EIBL et al.** *IOVS,* 2007
- *Ophthalmologe,* 2007
- **LÜKE et al.** *Exp Eye Res,* 2008
- **NISHI et al.** *J Cat Refract Surg,* 1996